# EUROPEAN PATENT APPLICATION

(11) **EP 1 772 463 A1**
(43) Date of publication of application: **11.04.2007**
(21) Application number: 05021808.0
(22) Date of filing: 06.10.2005
(51) Int. Cl.: C07K 14/47

(54) **Pleckstrin-based fusion protein and method for monitoring of enzyme activities by FRET**

(71) Applicant: EMBL, D-69117 Heidelberg (DE)
(72) Inventor: Schultz, Carsten, 69117 Heidelberg (DE); Brumbaugh, Justin, 53705 Madison (US); Schleifenbaum, Andreas, 2517 EM Den Haag (NL)
(74) Representative: Reitstötter - Kinzebach

(57) **Abstract**

The invention relates to a fusion protein comprising two fluorophores, a pleckstrin PH domain, a pleckstrin DEP domain and at least one substrate region for an enzyme performing a covalent modification, wherein the pleckstrin PH domain and the pleckstrin DEP domain are located between the fluorophores, and the substrate region is located between one of the two pleckstrin domains and the fluorophor on its side. It also relates to a method for determination of the activity of at least one enzyme performing covalent modifications of a polypeptide using said fusion protein and determination of FRET between the two fluorophores, and to the use of said fusion protein in such a method. It further relates to a nucleic acid encoding said fusion protein, to a cell expressing said fusion protein, to a transgenic multicellular non-human organism (animal or plant) expressing said fusion proteins, and to a kit for performing the method.

## Description

The invention relates to fusion proteins comprising two fluorophores, a pleckstrin PH domain, a pleckstrin DEP domain and at least one substrate region for an enzyme performing a covalent modification. It also relates to methods for determination of the activity of enzymes performing covalent modifications of a polypeptide by determining FRET, and to the use of said fusion protein for determination of the activity of enzymes performing covalent modifications of a polypeptide. It further relates to nucleic acids encoding said fusion proteins, to cells expressing said fusion proteins, to transgenic multicellular non-human organisms (animal or plant) expressing said fusion proteins, and to kits for performing the methods.

In contrast to the well-known situation in prokaryotic model organisms, where external stimuli and internal states are usually directly transformed into changes in the gene expression pattern, the ability of eukaryotic cells to act and to react is largely a matter of biochemical information processing performed by signalling cascades involving both membrane-bound and cytoplasmatic molecules. This enables eukaryotic cells to display much more complex behaviour, with a large number of external and internal signals being logically summated to finally result in a reaction which may or may not involve changes in the gene expression pattern and/or changes in the activities of pre-existing, cytosolic or membrane-bound elements, including, among others, the effectors for cell motility, phagocytosis, release of granules and, ultimately, cell cycle control.

In recent years, for many of the most active and complex cell types of the human and animal body intricate networks of signalling cascades have been unveiled. These networks are generally based upon a cascade of covalent modifications, where the primary stimulus, often initially sensed in terms of a change in aggregation or conformation of the primary sensor, is converted into a covalent modification of some target protein, usually by phosphorylation/dephosphorylation of crucial hydroxyl-containing amino acid residues of the latter, which is activated thereby and in turn becomes a modifying enzyme for another target protein or set of target proteins, until eventually effector proteins are activated or inactivated, transcription factors modified, and/or chromatin structures changed. These cascades may branch into different pathways leading to distinct effects, but they may also, and most interestingly, converge. In the latter case, an individual protein is susceptible to different types of modification and/or other stimuli, and the ensuing degree of activity is a function whose parameters are these modifications and stimuli. In general, this biochemical summation of signals provides equivalents to the fundamental logical operators "and", "or" and "not", thereby allowing for a highly sophisticated meshwork of information-processing elements.

In recent years, it has become increasingly clear that this network and its proper functioning are essential to all elements of human and animal health, from physiological development to pathological states. Some prominent examples of organs where malfunction of cellular information processing is thought to be especially detrimental comprise the nervous and the immune system.

Currently, many elements of these networks have been identified, and further will be in the near future. However, it is evident that understanding of the entire information-processing network will require detailed knowledge not only of the identities of its members but also of their interactions, especially the quantitative aspects of these interactions. It is indispensable to understand not only which component may interact with which other (a question partly to be answered by conventional protein interaction studies and probably, in the near future, molecular modelling), but also which component actually, under a given set of conditions, does interact with which other, and also to which degree it does so, preferably also taking into account the temporal aspects of information processing. The importance of rhythms and oscillations in biological systems is generally well established by now, and it can be expected that cellular information processing will be involved here too. But it is also mostly the temporal aspects of information processing which, for example, may make the difference between a valuable analgesic and an addictive drug.

However, so far the unravelling of these paths and networks has been seriously hampered by a deficiency in methodology. Tools for studying covalent modifications of candidate proteins have been available for some time and are known to the person skilled in the art, but these methods are generally tedious, resource-consuming and far too slow in the actual performance to allow for resolution of the more subtle aspects of cellular information processing. Typically, they require the use of dual antibodies - one directed against the candidate molecule and one against phosphorylated amino acids - and the solubilization of an isolated tissue sample, from which the candidate protein is isolated using the former, and assayed for relevant phosphorylation using the latter antibody. The assay itself may be implemented as, for example, a Western blot or immunosorbent assay. Such procedures are known in the art.

Apart from the fact that it may be difficult, and is generally tedious and expensive, to obtain a suitable pair of antibodies, the laborious process makes it virtually impossible to achieve fine temporal resolution and is not well suited for large test series as required, for example, in modem pharmacology. Reliable and reproducible quantification of the results may also be problematic. Moreover, the need for extraction precludes re-usage of a sample to assay the effects of sequential stimuli (sometimes an experimental animal may have to be killed in order to obtain a single sample, especially when solid tissues are of interest), and above all, it is not suitable for observation on the single-cell level, which becomes increasingly desirable as more and more complex organs, in which cells of different types cooperate, are targeted by researchers. Thus, there exists a need for a technique that allows for the study of covalent protein modifications *in situ,* preferably with single-cell resolution, and in real-time.

In general, light microscopy is the approach of choice of single-cell real-time *in situ* measurements whenever applicable. Over the last decades, advances in the science of fluorescent substances have greatly increased the applicability of light microscopy to subcellular structures and events; most notably, they have allowed the phenomenon of fluorescence-resonance energy transfer ("FRET"), first postulated, on a purely theoretical basis, by Förster in 1948 and described in more detail by Matyus 1992 (J. Photochem. Photobiol. B. 13;12(4):323-37), to be successfully employed in the study of molecular interactions, which are in themselves well below the resolution limit of light microscopy. The use of FRET for the study of living cells is thus generally known in the art.

Basically, FRET is a form of radiation-free energy transfer between two fluorescent groups ("fluorophores") with different but overlapping spectra that occurs when they are in sufficient proximity for their dipole moments to interact by resonance.

The borderline for the occurrence of FRET is rather sharp, the amount of energy transferred being inversely correlated to the sixth power of the distance between the fluorophores; the critical distance ("Förster distance") for FRET to occur depends on the chemical nature and sterical orientation of the fluorophores, but is usually in the dimension of about 10 nm, well suited to study interactions of complex biomolecules. In general, the Förster distance must be determined empirically for any given approach.

In order to be suitable for *in situ* experiments, the native protein must contain fluorophores in working order, without the need for any staining or dyeing procedures. In 1996 Mitra and co-workers (Gene 173(1 Spec No):13-7) first demonstrated the feasibility of FRET between two fluorescent proteins derived from the cnidarian *Aequorea victoria.* Their use for real-time observations was described in 1999 by Periasamy & Day (Methods Cell Biol. 58:293-314). Thus, FRET analysis of fluorescent proteins is basically known in the art.

With a wide range of fluorescent proteins now being available, which may be used for the construction of fusion proteins and the generation of transgenic organisms expressing the same, the applicability of FRET to the study of "*trans*-interactions" - herein used to denote interactions which either occur between different molecules or involve physically distinct elements of a large macromolecule freely mobile to each other - is feasible. Approaches making use of fluorescent proteins for the study of intermolecular and/or interdomain interactions have been described extensively in the art.

An exemplary application of FRET for the study of *trans*-interactions is described in WO02/090987. Here, two entities that are normally separate molecules are combined to form a fusion protein. Each of these entities is closely and rather rigidly linked to one of two fluorophores, and the two entity/fluorophore blocks are loosely connected by means of a flexible, amorphous linker structure essentially consisting of the widely-used [G₃S]ₙ structure. Thus, anything that promotes the association of the two entities will also enhance FRET between the two fluorophores, and vice versa.

However, FRET may also be used to determine events which are "cis-active", used herein to denote processes which involve conformation change within a single molecule. This contrasts with the measurement of *trans*-active events in that the factor influenced and to be measured is the conformation of a single entity, rather than the mutual affinity of two entities.

The use of FRET for the measurement of cis-active processes has also been described in the art. The best-known example is represented by the "cameleon" (sic) by Miyawaki and colleagues (Nature, 388(6645):834-5, 1997), a protein which is basically calmodulin with two fluorophores attached (usually implemented as a tripartite fusion protein). Calmodulin has long been known to change its conformation depending on Ca⁺⁺ levels, thus the cameleon's FRET activity may be used to indirectly assess intracellular Ca⁺⁺ levels *in situ* and in real time.

As covalent modifications of a signalling cascade protein are thought to exert their effects on the protein's activity by inducing changes in the protein's conformation, the idea being that the presence of additional charged and/or sterically bulky groups will warp the conformation to the point where the mutual repulsion of the groups is balanced by the increasing strain of the rest of the molecule. Thus, FRET measurements of modifiable protein domains flanked by fluorophores open intriguing possibilities for single-cell real-time *in situ* measurements of changes in activation levels.

Based upon the fact that most modifying enzymes are, and must be due to the necessities posed by the existence of a network of information transfer pathways and the concomitant plurality of proteins to be modified, specific for rather short motifs ("target sequences" or "substrate sequences"), the essential is a structure where a protein comprises a target sequence and fluorophores in such an arrangement that covalent modification of the target sequence changes the spatial arrangement of the fluorophores.

FRET probes based on proteins whose conformation undergoes shifts in reaction to covalent modification have been described in the art. Schleifenbaum and co-workers describe (J.Am.Chem.Soc 126(38):11786-11787, 2004) a fusion protein based upon pleckstrin which is suitable for measuring the activity of protein kinase C (PKC), often considered as the archetypical signal processing enzyme. This protein, in which phosphorylation of a domain (such domains or motifs will herein be referred to, functionally, as the "signal acceptor regions" or, structurally, "substrate regions" of a protein) interspersed between the two fluorophores causes a conformational shift leading to an increase in FRET efficiency, was named KCP-1. KCP-1 is characterized by the fact that it uses the PH and DEP domains of pleckstrin, enclosing the substrate region between them. This corresponds to the natural structure of pleckstrin, which bears a single PKC-responsive signal acceptor site between its PH and DEP domains.

However, the use of FRET probes based on naturally occurring proteins whose conformation undergoes shifts in reaction to covalent modification is largely restricted to the measurement of those enzymes which influence the underlying proteins themselves; attempts to disentangle the substrate region from the rest of the molecule in order to obtain FRET probes with more general applicability have failed so far. Accordingly, it was found that the KCP-1 probe is less versatile than expected, its substrate region not being suitable for replacement with arbitrary sequences. Thus there is an unmet need for further FRET probe proteins which are suitable for assessing a wider range of enzymes, their substrate regions not being restricted to the natural substrate sequences of the underlying conformation ally variable proteins.

Furthermore, the high complexity of the information processing networks makes it desirable to be able to measure more than a single enzyme's activity. An obvious approach consists in simply co-expressing differently coloured FRET probes for different enzymes (e.g. a blue to cyan and green one for enzyme X and a yellow to orange and red one for enzyme Y) in what is termed "multi-channel FRET". As said before, most fluorescent proteins in use today belong to one of two lines, each comprising a number of differently-coloured (i.e. possessing different excitation and/or emission spectra) but otherwise almost indistinguishable proteins; it is therefore usually possible to exchange a given one for a different one, no changes in molecule shape or Forster distance resulting.

In reality, though, the feasibility of this multi-channel approach is severely limited by a number of factors. Firstly, the exact determination of a larger number of fluorescence intensities poses significant technological problems, as the emitted light will have to pass through a complex system of optical filters in order to separately determine the values of each channel. This in turn reduces the available signal intensity, making necessary stronger fluorescence, which can only (and within certain limits) be achieved by more powerful excitation, which again will finally be detrimental both to the sample and to the fluorophores themselves, which are prone to be subject to "photobleaching", i.e. irreversible loss of their fluorescent properties due to side-reactions induced by energetic overcharge. Moreover, individual fluorescence emissions are not monochromatic but rather tend to be complex spectra, so proper separation of them may be difficult. This holds true especially in a biological system, where autofluorescence of other cellular components (most notoriously flavoproteins) is often uncomfortably high. When the proteins to be studied are suspected to be arranged in multiprotein complexes, their close packing may even offer opportunity for intermolecular FRET between different probes. Above all, it should be kept in mind that any probe protein used to investigate information processing actually acts as a parasite upon the signalling cascade it is involved in, by accepting input but not generating any output, which may cause major disturbances in the signalling processes themselves and thus lead to observational artefacts if expression of the probe is unduly high. Moreover, the presence of certain substrate domains has been demonstrated to exert a negative feedback regulation on the activity of the modifying enzymes. Therefore, it is generally advisable to use as few types of probes and corresponding channels as possible. In addition, when working with multicellular organisms, the generation of multiple transgenes poses additional difficulties.

Thus, we have a paucity of FRET channels which is awkward with regard to the complexity of the networks to be studied, and it would be advantageous to be able to use a given FRET channel for the determination of more than one enzyme activity.

Of course, when the disadvantages of multi-channel FRET do not render this approach unacceptable, the possibility of measuring two enzyme activities per FRET channel still offers the additional benefit of potentially doubling the number of activities which can be measured per assay. Alternatively, the experiment may be designed to use redundancy for higher resolution or reliability.

Thus, one object of the present invention was to provide further and more versatile proteins for FRET-based determination of the activity of enzymes which covalently modify other polypeptides. In another aspect, the object of the present invention was to provide a probe protein and method to its application which is able to determine activities of two or more enzymes using one single FRET channel ("dual probe protein" or "multiple probe protein").

Surprisingly, it was found that the architecture of pleckstrin-based fusion proteins allows for the incorporation of a signal acceptor region between one of the two pleckstrin domains and the fluorophor on its side, in combination with or instead of the signal acceptor region between the PH and DEP domains. Herein, the signal acceptor region between the PH and DEP domains will be referred to as the "internal" or "additional", the signal acceptor region between one of the two pleckstrin domains and the fluorophor on its side as the "external" or "mandatory" signal acceptor or substrate region.

Thus, in one aspect the present invention relates to a fusion protein comprising two fluorophores, a pleckstrin PH domain, a pleckstrin DEP domain and at least one substrate region for an enzyme performing a covalent modification, wherein the pleckstrin PH domain and the pleckstrin DEP domain are located between the fluorophores, and the substrate region is located between one of the two pleckstrin domains and the fluorophor on its side ("external" or "mandatory" substrate region).

As used herein, a "fusion protein" is a recombinant protein in which at least two or more amino acid sequences, preferably distinct functional units, from different proteins have been joined, preferably a recombinant protein which has been constructed by a process comprising recombination of at least two different nucleic acid sequences.

As used herein, a "recombinant protein" is a protein whose sequence as a whole lacks marked homology with any naturally occurring protein and/or whose structure and/or physical properties are essentially different from those of any naturally occurring protein, and/or whose functional properties are different from those of any naturally occurring protein. In particular, a recombinant protein may be a fusion protein.

As used herein, a "fluorophore" is a distinct chemical structure which is capable of producing a significant amount of fluorescence without the need for other auxiliary or supporting structures.

In FRET, energy transfer generally occurs from the lower wavelength fluorophore to the higher wavelength fluorophore, the latter ("acceptor") emitting light according to its own spectrum at the cost of the output of the former ("donor"), which is reduced but not influenced qualitatively in terms of wavelengths. In an exemplary arrangement, the donor exhibits blue-to-green fluorescence (meaning that is absorption is highest for blue light, and its emission peaks at green light) and the acceptor green-to-yellow; when FRET occurs, irradiation with blue light will result in yellow instead of green fluorescence, allowing the degree of interaction of the two fluorophores to be determined by simply measuring the ratio of yellow to green light.

Two widely used lines of fluorescent proteins are derived from proteins originally isolated from different taxa of cnidarians (*Aequorea victoria,* mentioned above, and *Discosoma* sp., the latter providing fluorescence in the range from yellow to far red). In these, the fluorophores are formed posttranslationally from a small number of adjacent amino acid residues in the interior of the molecule. In consequence, fluorescence of these proteins is largely independent of the proteins' environment, and they can be easily fitted into fusion proteins. Additionally, by modification of the amino acid sequence a large number of mutants with different spectra were obtained, which are invaluable as FRET generally requires two fluorophores with different but overlapping fluorescence spectra. As an additional benefit, the spatial structures of these proteins are virtually identical within their line, so exchange of a fluorophore for a differently coloured one has no effects on sterical effects and/or the Förster distance.

As used herein, "different fluorescence spectra" are spectra whose absorption (excitation) spectra are different from each other, and whose emission spectra are different from each other. They may still be "overlapping fluorescence spectra", meaning that the emission spectrum of one fluorophor may be similar to the absorption spectrum of the other.

Suitable fluorophores are known in the art. It is preferred if they are selected from the range of fluorescent proteins derived from the green fluorescent protein of the jellyfish *Aequorea victoria,* known as "GFP". In particular, these proteins include, but are not limited to, Blue Fluorescent Protein (BFP), Cyan Fluorescent Protein (CFP), Green Fluorescent Protein (GFP) itself, in particular GFP² having the amino acid sequence encoded by the nucleotide sequence SEQ ID NO:4, and Yellow or Yellowish-Green Fluorescent Protein (YFP), in particular EYFP having the amino acid sequence encoded by the nucleotide sequence SEQ ID NO:5, as well as a number of derivatives with equivalent or improved specific extinction and stability. It is also preferred if they are selected from the generally higher wavelength (yellow to far red) range of fluorescent proteins derived from the red fluorescent protein of the anthozoon *Discosoma* sp., known as "RFP".

As used herein, the term "pleckstrin" refers to the human protein named pleckstrin, GeneBank accession number NP_002655 / gi:4505879, or any sequence identical or markedly homologous thereto. Pleckstrin was first described by Tyers and co-workers in 1988 as the major substrate for protein kinase C in thrombocytes (Nature 333(6172):470-473 and J. Cell Biochem. 40(2):133-145), the phosphorylation sites were identified by Abrams and co-workers in 1995 (J. Biol. Chem. 270(40):23317-23321), but its three-dimensional structure has not been determined at high resolution yet.

As used herein, the term "PH domain" refers to the more aminoterminally located of the two *p*leckstrin homology domains found in pleckstrin, extending from arginine-7 to alanine-99 inclusive, or any sequence identical or markedly homologous thereto, in particular any sequence that is capable of interacting with the interaction partners of PH.

As used herein, the term "DEP domain" refers to the "*D*ishevelled, *E*gl-10 and *P*leckstrin" domain found in pleckstrin, extending from threonine-136 to aspartate-221 inclusive, or any sequence identical or markedly homologous thereto, in particular any sequence that is capable of interacting with the interaction partners of DEP.

As used herein, the term "markedly homologous" refers to an amino acid sequence whose sequence shows no more than 20%. preferably no more than 10%, more preferably nor more than 5% difference according to the Needleman-Wunsch algorithm, or which shows no more than 20%, preferably no more than 10%, more preferably nor more than 5% difference according to the Needleman-Wunsch algorithm using the above parameters when disregarding conservative amino acid substitutions such as substitution of one amino acid for another of the same class (e.g. leucine for isoleucine, arginine for lysine, etc.) and such as may be attributed to obvious sequencing errors (e.g. alleged glycine to glutamate shift by misconstruing GGG for GAG in a single-read), or which shows no more than 20%, preferably no more than 10%, more preferably nor more than 5% difference according to the Needleman-Wunsch algorithm using the above parameters when disregarding conservative amino acid substitutions, obvious sequencing errors and non-conservative amino acid substitutions, insertions and deletions located at positions of the amino acid sequence which do not destroy or significantly alter the structural properties, biological activity and/or applicability of the protein.

As used here, a sequence is considered as "identical" to another if their sequences are of the same length and do not contain any mismatches that cannot be attributed to sequencing errors.

As used herein, the term "enzyme", unless stated otherwise, refers to enzymes performing covalent modifications on other polypeptides, especially as part of a signal transmission cascade and/or signal-processing network.

As used herein, a "covalent chemical modification" or "covalent modification" of a protein may be basically any change that involves the formation or cleavage of a covalent bond, but unless noted otherwise, it is to be understood here that it means a modification characterized by the addition or removal of a small chemical group to or from any of the protein's amino acid residues. In particular, covalent modifications comprise esterifications and, less frequently, etherifications of serine, threonine and tyrosine residues and their dissolutions, especially the phosphorylation and dephosphorylation of such residues. Generally, such covalent modifications are mediated by pairs of enzymes, e.g. kinases for phosphorylation and phosphatases for dephosphorylation. Obviously, the activity of both members of a pair must be strictly regulated to attain reliable information processing, but they may be subject to different influences, thereby also contributing to the information summation process.

Expediently, the fusion proteins of the invention are such that the covalent modification of the substrate region induces a conformational change of the protein.

As used herein, the term "polypeptide" may denote any chain of amino acids, regardless of length or post-translational modifications.

As used herein, the term "conformation" refers to the spatial arrangement which is energetically most favourable for the molecule or domain of interest under a given set of conditions, and which the molecule or domain will therefore assume under these conditions unless subjected to external forces. A "conformational change" is thus a shift in the shape of the molecule or domain which is energetically favourable and will prevail when the molecule or domain is not subjected to external forces. In accordance with the present invention, said conformational change causes a detectable change in FRET between the fluorophores.

As used herein, the term "substrate region" denotes any part of a protein that may be covalently modified by specific interactions of the protein with a suitable enzyme. Preferably, the substrate regions of the fusion protein according to the invention are selected from the substrates of protein kinase A, more preferably from the protein kinase A consensus sequences, in particular the Kemptide sequence (LRRASLG) or any subset or derivative thereof which can be phosphorylated by protein kinase A; from the substrates of protein kinase C, more preferably the amino acids from isoleucine-100 to aspartate-135 inclusive of human pleckstrin or any subset or derivative thereof which can be phosphorylated by protein kinase C; and from histones, preferably from amino acids 1-122 of histone H3 tail, amino acids 1-92 of histone H4 tail, amino acids 1-120 of histone H2A or amino acids 1-120 of histone H2B, or any subset or derivative thereof which can be phosphorylated, acetylated or methylated. Further suitable substrate regions are those which are susceptible of being covalently modified by one or more of the following enzymes: ERK, MAPK, Titin kinase, Aurora A, and Aurora B.

As used herein, the term "side" refers to that part of a protein which extends from the reference point to the terminus of the protein. When reference is made to the "side of a pleckstrin domain", it is to be understood that of the two parts on the aminoterminal and carboxyterminal side of the domain in consideration this denotes the part of the molecule which does not comprise the other pleckstrin domain. Thus, when PH is located aminoterminally to DEP, the "PH side" is the part of the protein from the amino terminus to the PH domain, and the "DEP side" is the part of the protein from the DEP domain to the carboxyl terminus; in other words, the overall architecture of the protein is essentially tripartite, with a PH side, a middle region comprising the PH and DEP domains and anything interspersed between them, and a DEP side.

With regard to the two pleckstrin domains, the fluorophor which is not separated from one by the other pleckstrin domain will be referred to as the fluorophor on the side of the respective pleckstrin domain. As regards the fluorophores, the donor may be located either on the aminoterminal and the acceptor on the carboxyterminal side of the region enclosed between them, or vice versa. In a particular embodiment of the invention, the donor is located on the carboxyterminal and the acceptor on the aminoterminal side of the region enclosed between them.

In a preferred embodiment of the invention, the DEP domain is located on the carboxyterminal side of the PH domain of the fusion protein, with or without additional sequences interspersed between these two domains. In this case, the fluorophor referred to as being "on the side of the DEP domain" will of course be the one located on the carboxyterminal side of the DEP domain, and the fluorophor "on the side of the PH domain" will be the one on the aminoterminal side of the PH domain, the presence or absence of any further sequences between the respective pleckstrin domain and the fluorophor notwithstanding.

It is especially preferred if at least one external substrate region is located between the DEP domain and the fluorophor on its side, and more preferred if one external substrate region is located between the DEP domain and the fluorophor on its side. If there is more than one external substrate region present, the effects of their modifications will be cooperative.

According to a particular embodiment, the region between the DEP domain and the external substrate region comprises a negatively charged sequence motif, preferably a negatively charged stretch of 8 to 20 amino acids and in particular of 10 to 15 amino acids, which stretch comprises 4 to 8, preferably 5 to 7 and in particular six negatively charged amino acids, most preferably a loop comprising the amino acid sequence EENSSDDDVILKE, interspersed between the DEP domain and the most proximal external substrate region on its side.

According to another embodiment, at least one external substrate region is located between the PH domain and the fluorophor on its side, and more preferred if one external substrate region is located between the PH domain and the fluorophor on its side.

In a preferred embodiment of the invention, the external (mandatory) substrate region of the fusion protein may be such as can be phosphorylated by protein kinase A. It is especially preferred if the external substrate region contains a protein kinase A consensus sequence, in particular the Kemptide sequence (LRRASLG) or any subset or derivative thereof which can be phosphorylated by protein kinase A. Protein kinase A is an important link in the intracellular signal transmission chain well known to those skilled in the art.

In another preferred embodiment of the invention, there is another substrate region, herein denoted the "internal" substrate region or signal acceptor, located in between the PH and the DEP domain of the fusion protein ("dual probe protein").

It is especially preferred if the internal (additional) substrate region is suitable for phosphorylation by protein kinase C as defined above. It is especially preferred if the internal substrate region contains a protein kinase C consensus sequence, in particular the amino acids from isoleucine-1 00 to aspartate-135 inclusive of human pleckstrin or any subset or derivative thereof which can be phosphorylated by protein kinase C. Protein kinase C, occurring in a considerable number of isoforms, is an important link in the intracellular signal transmission chain well known to those skilled in the art.

In another preferred embodiment of the invention, there is no other substrate region located in between the PH and the DEP domain of the fusion protein, i.e. the fusion protein possesses only the external but no internal substrate regions. In lieu of the internal substrate region, the PH and the DEP domain are connected by a linker. A suitable linker may, for instance, comprise a sequence that is derived from the corresponding portion of the native pleckstrin molecule, preferably the amino acids from isoleucine-100 to aspartate-135 inclusive, with the amino acids acting as substrates for modification in native pleckstrin replaced by such as cannot be modified by PKC. It is particularly preferred if the modifiable amino acids serine-113, threonine-114 and serine-117 have been replaced with amino acids selected from alanine or glutamate.

In a particular embodiment of the invention, the fluorophores of the fusion protein are those of green and yellow fluorescent protein (GFP and YFP), or proteins derived therefrom which possess equivalent excitation and emission spectra.

According to a particular embodiment of the present invention, the fusion protein has the amino acid sequence encoded by the nucleotide sequence SEQ ID NO:1 (KAP-1), SEQ ID NO: 2 (KAP-2) or SEQ ID NO: 3 (KCAP-1), or any sequence bearing a marked homology with any of the said sequences or a functional equivalent thereof.

The internal and external signal acceptor are independent of each other, allowing for the determination of unrelated enzyme activities, and they produce distinguishable signals within the same FRET channel. Overlapping effects (i.e. distortion of results due to simultaneous modification of both acceptors) may be separated by proper factorial design of the experiment and/or quenching of one of the two pathways in question by addition of a suitable inhibitor. Different isoenzymes may also be selectively inhibited to identify the active one.

The fusion proteins termed KAP-1 and KAP-2 possess only the external substrate region. It was demonstrated that the external region is more versatile in terms of the sequences to be used therein; e.g. it was shown that the external region may contain a substrate region phosphorylated by protein kinase A (PKA), which was not compatible with the internal region, which imposes more severe limitations on the nature of the substrate sequence. Moreover, in some cases, where the enzyme is usually highly studded with covalently attached groups and the signal is essentially transmitted as a detachment of some of these, the external signal acceptor, which transforms maximal phosphorylation into minimal FRET, may be experimentally advantageous even when only a single enzyme of interest is to be studied.

In the protein designated "KCAP-1 ", covalent modification of the external signal acceptor leads to a decrease rather than an increase in FRET efficiency.

Without being limited by theory, it is thought that modification of the internal region induces a splaying of the PH and DEP domains which pulls the fluorophores into closer vicinity, the splaying allowing a negatively charged region on the carboxyterminal side of the DEP domain to interact with the PH domain. By contrast, modification of the external acceptor is assumed to bend the carboxyterminal parts of the molecule away from the rest, thereby increasing the distance between the fluorophores. The latter effect is considered as generic and not restricted to any particular type of modification as long as the net charge of the external acceptor is influenced by the modification. Such modifications comprise, among others, phosphorylation/dephosphorylation and acetylation/deacetylation.

In another aspect, the present invention relates to a nucleic acid encoding a fusion protein according to the invention. It is especially preferred if the nucleic acid is DNA, and more preferred if it is double-stranded DNA ("dsDNA"). It is most preferred if it is inserted into a plasmid, especially a plasmid comprising elements required for expression in eukaryotic cells, in particular the eukaryotic cells of interest, or other system providing the ability for expression. Appropriate elements (promoters, enhancers, terminators, splice site donors, etc.) are known to one skilled in the art, as are the tools and methods required for management and handling of DNA molecules.

In a particularly preferred embodiment, all the proteins to be used are encoded by a physically contiguous piece of DNA, especially a single plasmid. In all embodiments, the sequence of the nucleic acid itself may be contiguous, or it may be interrupted by introns and/or intron-like elements, provided that transcription and translation will ultimately result in an amino acid sequence which corresponds to a protein according to the invention. The sequence may also exist in the form of a number of physically disjoint portions, e.g. produced by enzymatic treatment ("restriction fragments"), which may again be joined to other molecules (e.g. in a plasmid or phage library), as long as it is still possible to reconstruct a sequence which, when transcribed and translated, results in an amino acid sequence which corresponds to a protein according to the invention.

It is also especially preferred if the nucleic acid is RNA, which may be single-stranded or double-stranded RNA. Methods for the handling of RNA are also described in the art. In a particular embodiment, the RNA is part of a viral vector system, for example a retroviral or togaviral vector system, as described in the art.

In another aspect, the present invention relates to a cell expressing a fusion protein according to the invention.

As used herein, the term "expression" is used to denote the presence of a detectable amount of the fusion protein within the cell, preferably presence of the fusion protein resulting from translation of an mRNA within the cell encoding the amino acid sequence of the fusion protein, more preferably presence of the fusion protein resulting from transcription of DNA within the cell and translation of the resulting mRNA.

In a preferred embodiment, the cell is a cell of a multicellular organism including plants, animals and humans, preferably a cell freshly isolated from a multicellular organism (primary cell) or isolated and expanded by application of proper stimuli but not immortalized (oligoclonal cell), or also preferably a cell belonging to an established cell line originally derived from a multicellular organism. It is particularly preferred if the established cell line is of primate or rodent origin, and most particularly preferred if the established cell line is selected from CV-1, COS, HeLa, 293T and CHO cells. Established cell lines and methods for the isolation of primary cells and their expansion into oligoclonal cells are generally known to one skilled in the art.

In one embodiment of the cell, expression of the fusion protein in the isolated or established cell of a multicellular organism is transient. It is especially preferred if the nucleic acid encoding the fusion protein is maintained episomally, and more preferred if the cell/vector system supports the amplification of the nucleic acid from which the fusion protein is expressed. In another embodiment of the cell, expression of the fusion protein in the isolated or established cell of a multicellular organism is stable, i.e. the transgene is permanently integrated into the genome. Here it is especially preferred if cells expressing the fusion protein are positively selected, and more preferred if the selection process employs the fluorescence of the fusion proteins themselves as selective criterion. In yet another embodiment of the cell, the cell is a primary or oligoclonal cell isolated from a transgenic multicellular organism. When the cells are stably transfected or isolated from a transgenic multicellular organism, it is preferred that their expression can be regulated. Appropriate expression control systems have been described in the art.

In a particular embodiment, the isolated cell of a multicellular organism, or the cell of a non-human multicellular organism *in situ,* is infected with a virus. Where the cells are transiently transfected to express the fusion protein, virus infection may take place before, during or after the transfection reaction, preferably after the transfection reaction. Thus the invention also provides a method for determining the influence of viral infection on intracellular signal transmission pathways. In a particular embodiment, the virus is transgenic to express the fusion protein, thus obviating the need for a separate transfection step. It is especially preferred if the transgenic virus belongs to a group selected from papovaviridae, adenoviridae, herpesviridae, poxviridae and retroviridae. Viral variants suitable for the expression of non-viral proteins during infection have been described in the art.

In another preferred embodiment, the cell is an individual of a unicellular organism, preferably a protist, more preferably a protist belonging to a genus selected from *Tetrahymena, Plasmodium, Toxoplasma* and *Trypanosoma,* or preferably an unicellular alga, preferable an alga selected from *Chlorella* or *Poterioochromonas,* or preferably a yeast, more preferably a yeast belonging to a genus selected from *Saccharomyces, Schizosaccharomyces* and *Pichia.*

In yet another aspect, the present invention relates to a transgenic multicellular organism (non-human animal or plant/fungus) characterized by a stably maintained transgene, in which a protein according to the invention is expressed under the control of a promoter, in particular a tissue-specific, developmentally regulated or externally controlled promoter. Such regulated promoters have been described in the art.

In a preferred embodiment of the invention, the multicellular organism is a plant. It is especially preferred if the plant belongs to the angiosperms, and more preferred if the angiosperm belongs to the genus *Arabidopsis,* or if the plant belongs to an agriculturally useful taxon.

In another preferred embodiment of the invention, the multicellular organism is an animal. It is especially preferred if the animal is a nematode, and more preferred if the nematode belongs to the genus *Caenorhabditis.* It is also especially preferred if the animal is an insect, and more preferred if the insect belongs to the genus *Drosophila.* It is also especially preferred if the animal is a vertebrate, more preferably if the vertebrate is a fish, most preferably if the fish belongs to the genus *Danio* (syn. *Brachydanio*), or more preferably if the vertebrate is an amphibian, most preferably if the amphibian belongs to the genus *Xenopus,* or more preferably if the vertebrate is a mammal, most preferably if the mammal is a rodent, especially preferably if the rodent belongs to a genus selected from *Mus, Rattus* or *Cricetus.*

In another aspect, the present invention relates to a method for the determination of the activity of at least one enzyme performing covalent modifications by using a fusion protein of the invention and determining FRET between the fluorophores. In particular, such a method comprises: (i) providing a fusion protein of the invention, (ii) determining the baseline FRET activity of the fusion protein; (iii) contacting the fusion protein with a test sample; and (iv) determining any change in FRET activity thereby to determine the activity of the enzyme in the test sample.

According to another aspect, the present invention relates to a method for detecting the presence or absence of an enzyme in a test sample, which method comprises (i) providing a fusion protein of the invention wherein the substrate region of the fusion protein is capable of being covalently modified by the enzyme; (ii) determining the baseline FRET activity of the fusion protein; (iii) contacting the fusion protein with the test sample; and (iv) determining any change in FRET activity thereby to determine whether the test sample comprises the enzyme.

In a particular embodiment, the method may be used for detecting the presence or absence or determining of the activity of an enzyme which does not directly interact with the fusion protein, given that the enzyme in question is a part of a known signalling cascade or pathway that ultimately leads to modification of the fusion protein and that the intermediary members of the cascade or pathway are present and functional. In particular, the method may be used for measuring the strength or detecting the presence or absence of a non-covalent interaction or signal, given that the non-covalent interaction is a part of a known signalling cascade or pathway as described.

According to another aspect, the present invention relates to a method for identifying an enzyme inhibitor, which method comprises (i) providing a fusion protein of the invention wherein the substrate region of the fusion protein is capable of being covalently modified by the enzyme; (ii) determining the baseline FRET activity of the fusion protein; (iii) providing a test substance (inhibitor candidate) and contacting the fusion protein with the enzyme in the presence of a test substance; and (iv) determining any change in FRET activity thereby to determine whether the test substance is indeed an inhibitor of the enzyme.

In a particular embodiment, the method may be used for identifying an inhibitor for an enzyme which does not directly interact with the fusion protein, given that the enzyme in question is a part of a known signalling cascade or pathway that ultimately leads to modification of the fusion protein and that the intermediary members of the cascade or pathway are present and functional. In particular, the method may be used for identifying an inhibitor of a non-covalent interaction or signal, given that the non-covalent interaction is a part of a known signalling cascade or pathway as described.

In all these aspects, the fusion protein may be provided in the form of the fluorescent, modification-susceptible polypeptide or, as long as suitable provisions are made for the expression of the said polypeptide, in the form of a nucleic acid whose sequence encodes the same. For *in vitro* assays, it is preferred if the fusion protein is provided in form of the polypeptide; for *in vivo* assays, it is preferred if the fusion protein is provided in form of a nucleic acid capable of expressing the polypeptide within the cell or organism. It is to be understood here that unless explicitly stated to the contrary, all methods described here may be applied to fusion proteins provided in either way; thus, a suitable object for any of the methods described here may be an *in vitro* reaction mix, a unicellular organism, a non-human multicellular organism or any cell, tissue or body part of the latter, which may be *in situ,* isolated, detached or dissolved, or any isolated human cell. When the protein is provided *in vivo,* the measurements themselves may be performed after dissolving the cells or, more preferably, *in situ.*

As used herein, the term "baseline FRET activity" refers to the FRET activity of the fusion protein before exposure to any potentially directly or indirectly modifying influence or stimulus that may either increase or decrease the FRET activity observable. As described below, it is measured by determining the energy transfer between the two fluorophores before exposition to any stimulus. At baseline, the fusion protein may be in active or in resting stage, i.e. with or without covalently attached modifications, depending on the nature of the individual substrate region and the experimental protocol. Likewise, the "change in FRET activity" is determined by measuring FRET activity after, or for real-time measurements, during administration of the stimuli and relating it to the baseline activity.

As used herein, the term "contacting" refers to the establishment of a situation where the fusion protein is enabled to physically interact with potentially modifying enzymes. For *in vitro* assays, this is performed appropriately by mixing; for *in vivo* assays, by expressing the fusion protein within a cell of interest and then exposing the cell or nonhuman multicellular organism containing the same to the substance, influence or stimulus whose effects are to be determined.

It is especially preferred if the modification to be measured comprises phosphorylation/dephosphorylation and/or acetylation/deacetylation of amino acid residues.

According to a preferred embodiment, the method allows for a real-time determination of enzyme activity. As used here, "real-time" refers to an analytical or observational method that obtains data about the process or system to be observed without any significant delay, enabling the observer to conduct his experiment at an arbitrary temporal resolution, preferably at a temporal resolution that matches the speed of the events to be observed and thus yields additional information about the system. Characteristically, biochemical information processing within cells takes place in the second to minute range.

According to a preferred embodiment, the method allows for an *in situ* determination of enzyme activity. As used here, "in situ" denotes an analytical or observational method that is suitable for examining cells or tissues in their natural context and order, without the need for dissolving or modifying their original structures.

According to a preferred embodiment, the method allows for single cell level determination of enzyme activity. As used here, "single cell level" refers to an analytical or observational method that may be used to obtain differential result for individual cells, especially neighbouring cells within an organized tissue.

The determination of FRET usually comprises the determination of FRET activity or FRET efficiency. As used herein, the terms "FRET activity" or "FRET efficiency" denote the percentage of absorbed energy that is transferred from the donor to the acceptor; thus at 100% FRET efficiency no donor emission, at 0% no acceptor emission will be observed. Hence FRET efficiency is acceptor fluorescence divided by the sum of donor and acceptor fluorescence as measured. The time required for the energy transfer is in the picosecond range. Techniques and instrumentations for assessing FRET intensities in living samples have been described extensively in the art.

In a particular embodiment of the invention, the method comprises the use of a fusion protein which possesses two substrate regions, in particular both an internal and an external substrate region, thereby permitting the parallel determination of the activities of two enzymes using a single FRET channel.

As used herein, a "FRET channel" refers to a combination of one excitation wavelength with two emission wavelengths. It will be noted that each FRET channel requires two metrological channels, whose output values are then combined to yield a single result for this channel.

As used herein, "parallel determination" of enzyme activities refers to the same sample being suitable for the determination of more than one enzyme activity. Using this sample, the activities may be determined simultaneously or sequentially.

In a preferred embodiment of the invention, the fusion protein is expressed within a living cell, preferably a eukaryotic cell, to be observed (*in vivo*). Preferably, the fusion protein is provided in form of a nucleic acid encoding the same. The expression of the fusion protein may be stable or transient. Techniques and instrumentations for stably or transiently expressing foreign proteins in different types of cells have been described and are familiar to those skilled in the art.

In a preferred embodiment of the invention, the fusion protein does not display any marked homology with any protein naturally occurring in the cell of interest, with the exception of the fusion protein's substrate region. Thus, although the cells to be investigated may be basically of any type, it is particularly preferred if they do not belong to the haematopoietic lineage.

In a preferred embodiment of the invention, it is possible to optionally suppress alternative pathways by applying specific inhibitors. For most signal transmission enzymes identified so far, appropriate inhibitors are available. Such inhibitors as well as their modes of usage are familiar to one skilled in the art, and they are widely used to clarify phenomena including intracellular signal processing by suppressing individual lines of information transfer. When a specific inhibitor causes a decrease in the intensity of the phenomenon to be observed, compared to an untreated sample, the pathway blocked may be held to be essential. As the method according to the invention is suitable for *in situ* measurements of live cells, the comparison may be obtained by performing a pair of measurements before and after addition of the inhibitor, thereby greatly simplifying the process. Thus, a skilled person will be able to obtain, by means of factorial design, a large quantity of information with comparatively few experiments. Particularly preferred inhibitors comprise Gö6983, H-7 and staurosporine, all of which are known to inhibit some or all isoforms of PKC.

In a preferred embodiment of the invention, the enzyme whose activity is to be measured is protein kinase A, or a protein whose substrate is markedly homologous to that of protein kinase A. It is especially preferred if one of the enzymes is protein kinase A, or a protein whose substrate is markedly homologous to that of protein kinase A, and the other is protein kinase C, or a protein whose substrate is markedly homologous to that of protein kinase C.

In a preferred embodiment of the invention the method comprises the parallel use of more than one fusion protein according to the invention, the fluorophore combinations of these proteins being preferably different ("multi-channel FRET") so that the use of n fusion proteins and n FRET channels allows for the parallel determination of up to n enzyme activities. Alternatively, the number of enzymes to be determined may be less than n, the redundancy making it possible to gather information at higher precision, or with increased reliability. Here each fusion protein can be provided in the form of the protein or the encoding nucleic acid, as described above.

As used herein, the term "different fluorophore combinations" or "differently coloured fluorophore combinations" denotes fluorophore combinations which form different FRET channels, i.e. pairs of fluorophores where both emission spectra of each pair are distinct from the emission spectrum of any other fluorophore present, in particular where both the excitation spectrum and the two emission spectra of each pair are distinct from the excitation spectrum and the two emission spectra of any other pair present.

It is especially preferred if in this aspect the proteins used are dual probe proteins, so the total of enzyme activities to be determined using n fusion proteins and n FRET channels may be up to 2n. Alternatively, the number of enzymes to be determined may be less than 2n, the redundancy making it possible to gather information at higher precision, or with increased reliability. Here each fusion protein can be provided in the form of the protein or the encoding nucleic acid, as described above.

In a particular embodiment of the invention, the method comprises using the protein according to the invention *in vitro* by adding it to a suitable cell-free assay mixture, preferably an assay mixture containing the necessary cofactors (e.g. divalent cations) and donors of small detachable groups suitable for covalent modification of polypeptides (e.g. ATP). Preferably, the protein is used in concentrations high enough to obtain a clear fluorescence signal over the background fluorescence of the assay mixture, and more preferably, in a concentration which is between the detection limit thus set by autofluorescence and the concentration where fluorescence quenching and/or intermolecular FRET due to molecular crowding begins to distort signal intensity.

In another, preferred embodiment, the method comprises using the protein for *in vivo* determination of enzymatic activities. In this embodiment, the protein itself may be introduced into the cells of interest prior to measurement of the activities, or, more preferably, a nucleic acid encoding an amino acid sequence which corresponds to a protein according to the invention according to the invention is introduced into the cells and the protein then, prior to measurement of the activities, expressed from this nucleic acid, which may be basically any nucleic acid suitable for protein expression in the cells of interest. In a particular embodiment of the invention, the protein is used by introducing a nucleic acid encoding an amino acid sequence that corresponds to a protein according to the invention into a progenitor or ancestor of the cell or cells to be assessed and stably maintaining the nucleic acid.

In another aspect, the present invention relates to the use of a protein according to the invention for determination of the activity of enzymes performing covalent modifications of a polypeptide.

In another aspect, the present invention relates to a kit for performing the method according to the invention. The kit comprises at least a fusion protein and/or a nucleic acid encoding a fusion protein according to the invention. The kit may further comprise means for transferring the aforesaid nucleic acid into a living target cell (which may be isolated or in its natural context) and/or a means for determining FRET between the two fluorophores. Methods for the transfer of nucleic acids into target cells (transfection) include, among others, lipofection, calcium phosphate precipitation, electroporation and the use of "gene guns" and viral delivery systems. They are essentially familiar to one skilled in the art, and may be selected according to the cell type of interest.

In a preferred embodiment of the invention, the kit comprises each of the proteins and/or a nucleic acid sequence for each of the proteins to be employed in the measurement. The kit may further comprise a means for transferring the aforesaid nucleic acids, which are preferably ready for expression of the respective protein, into a living target cell (which may be isolated or in its natural context) and a method for determining the levels FRET between the two fluorophores of each of the proteins. In a particular embodiment, several or all of the sequences are contained within a single physically contiguous piece of nucleic acid. When more than one physically distinct piece of nucleic acid is used, the kit may comprise a method for transferring these individual pieces in parallel or sequential fashion. It is especially preferred if the nucleic acids are provided in the form of plasmids.

In a preferred embodiment of the invention, the kit also comprises a means for the transfection of target cells.

In a preferred embodiment of the invention, the kit also comprises inhibitors for the selective blocking of a signalling pathway, especially for the selective blocking of an alternative signalling pathways, as has been described above.

In a preferred embodiment of the invention, the kit comprises at least one fusion protein and/or a nucleic acid encoding at least one fusion protein selected from the amino acid sequences encoded by SEQ ID NO:1, SEQ ID NO:2 or SEQ ID NO:3, or any sequence bearing a marked homology with any of the said sequences, and at least one inhibitor for PKC.

The present invention will now be described in more detail with reference to the following examples and drawings which illustrate the invention.

In the drawings,

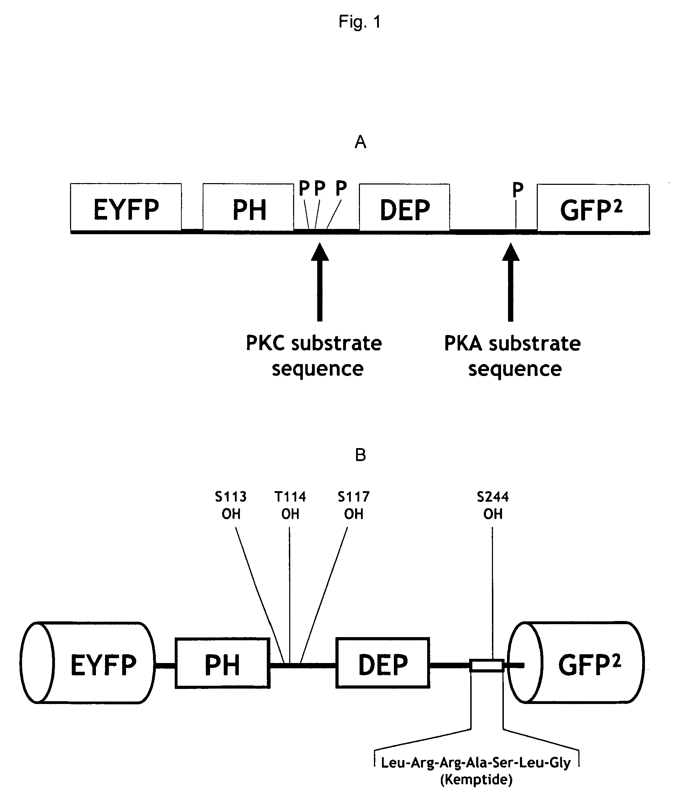

- Fig. 1: shows a particular design of fusion protein used in the invention, with Fig. 1 A and B giving the linear arrangement of the domains of a particular protein of the invention, KCAP-1;

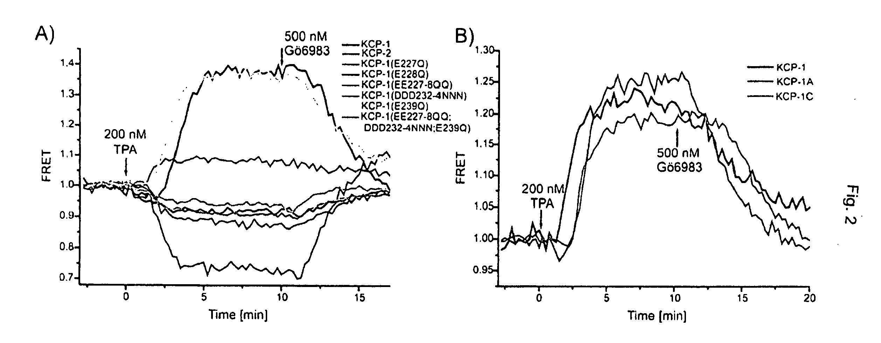

- Fig. 2: shows the effects of mutations in the negatively charged region carboxyterminally adjacent to the DEP domain of the protein (A: Constructs carrying mutations to acidic amino acids in KCP-1's C-terminal loop as described in table 3; B) Addition of amino acids between KCP-1's pleckstrin region and GFP2 as described in table 4; graphs are representative of at least three experiments; KCP-1 and KCP-2, fusion proteins with internal substrate region only (reference only); E227Q, glutamate 227 changed to glutamine; E228Q, glutamate 228 changed to glutamine; EEE2278QQ, both glutamate 227 and glutamate 228 changed to glutamines; DDD2325NNN, aspartate 232, aspartate 234 and aspartate 235 changed to asparagines; E239Q, glutamate 239 changed to glutamine);

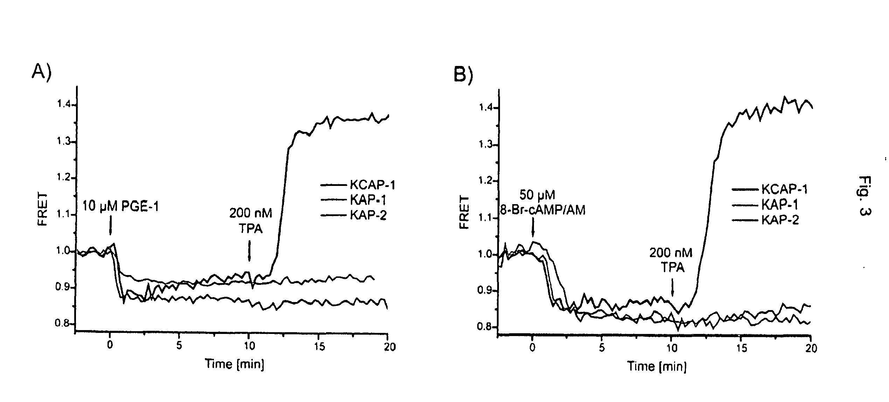

- Fig. 3: shows the changes in FRET observed when cells expressing KCAP-1, KAP-1, or KAP-2 are treated with PKA activators like A) the physiological stimulus, prostaglandin E-1 and B) the non-degradable cAMP analog 8-Br-cAMP/AM (PGE-1, prostaglandin E-1; 8-Bromo-adenosine-cyclic 3',5' monophosphate acetoxymethyl ester (8-Br-cAMP/AM); TPA, (tetradecanoylphorbolacetate));

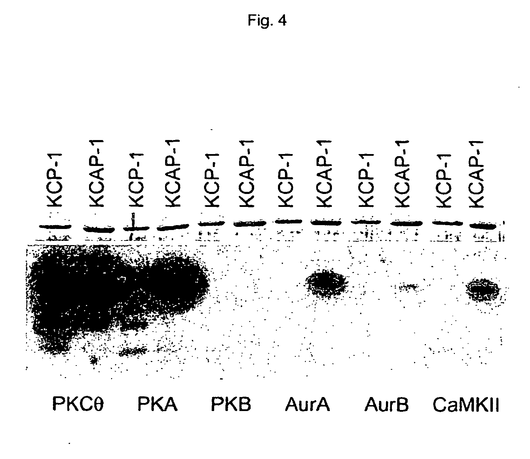

- Fig. 4: shows the *in vitro* phosphorylation of a reference protein KCP-1 and a fusion protein of the invention, KCAP-1, observed after treating the probes with various protein kinases *in vitro* (PKCθ, protein kinase Cθ; PKA, protein kinase A; PKB, protein kinase B; AurA, Aurora kinase A (aka Aurora-2, STK6, ARK1 or Aurora/IPL1-related kinase); AurB, Aurora kinase B (aka Aurora-1); CaMKII, Ca⁺⁺ calmodulin dependent kinase II);

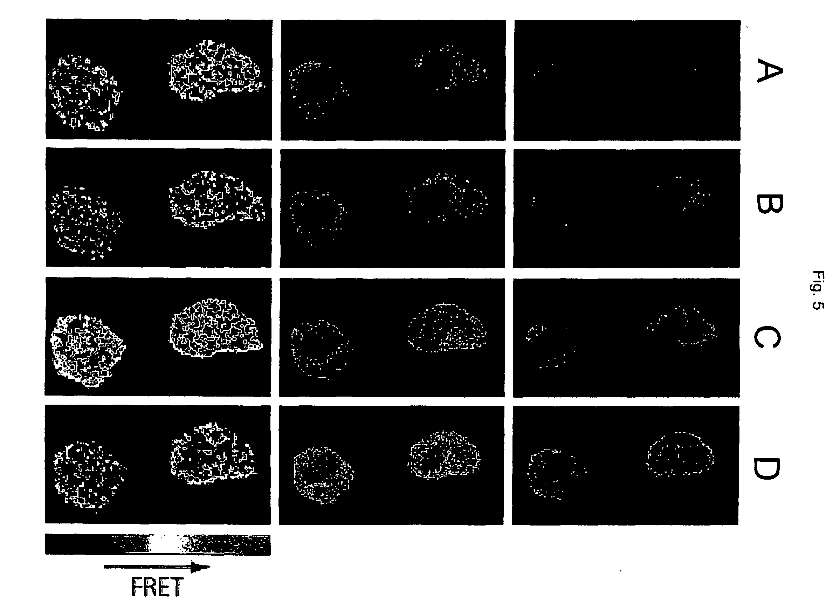

- Fig. 5: shows the results of a real-time *in situ* measurement of N1 E-115 human neuroblastoma cells expressing KCAP-1 (top row: GFP² emission; middle row: EYFP emission; bottom row: FRET ratio; from left to right, images captured at: (A) 0.5 minutes before or (B) 5 minutes after addition of 10 µM Forskolin / 100 µM IBMX, (C) 7.5 minutes after addition of 200 nM TPA, and (D) 7.5 minutes after addition of 500 nM Gö6983);

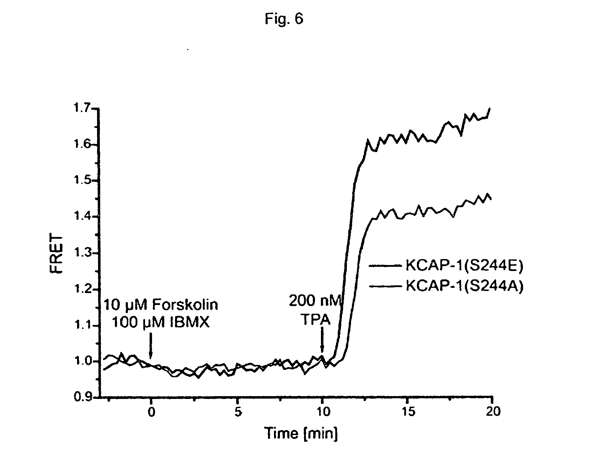

- Fig. 6: shows the changes in FRET observed when cells expressing KCAP-1 having mutations to Ser244 (the putative phosphoacceptor amino acid of KCAP-1's Kemptide sequence) are treated with PKA activators like forskolin/IBMX; graphs are representative of at least three experiments.

### Examples

### 1. Generation, expression and light microscopy detection of KCAP-1

KCP-1 (GeneBank AJ783754), as described in Schleifenbaum et al. 2004 (J.Am.Chem.Soc. 126(38): 11786-11787), was modified by PCR to include two Bbsl restriction sites immediately downstream from the plasmid's pleckstrin coding region.

Since Bbsl cuts outside of its recognition sequence, the fragment of DNA carrying the recognition sites, one on each strand of the DNA, was removed without disturbing KCP-1's original coding sequence. The resulting open plasmid had specific overhangs that were used to ligate DNA fragments of choice in-frame to quickly and specifically alter the amino acid sequence of KCP-1.

Briefly, PCR was used to amplify a 719 base pair fragment of KCP-1's pleckstrin coding region. The forward primer (5'- CCG GAG CCA TGG AAC CAA AG) included a 5' Ncol site while the reverse primer (5'-TCA TGG ATC CCA GTC TTC GTA TTA GGA AGA CCC TTC TTT CAG AAT CAC-3') harboured two Bbsl sites as well as a 3' BamHl site. The amplified DNA was cut with Ncol/BamHl and gel purified. The resulting fragments were sub-cloned into the pET-9d vector for amplification and sequence verification. The fragments were then cloned into the previously described pYG vector (as described in Supplementary Material of Schleifenbaum et al. 2004 (J.Am.Chem.Soc. 126(38): 11786-11787)). The resulting construct, called mKCP-1 (modified KCP-1), contained a 5' EYFP gene, followed by a pleckstrin coding region (codes for amino acids 1-239), an insert coding for two Bbsl cut sites and a 3' GFP² gene.

The dual parameter kinase sensor for PKC and PKA was constructed by inserting DNA that codes for a preferred PKA substrate sequence, Kemptide (amino acid sequence LRRASLG) into mKCP-1 opened with Bbsl. To create the double stranded Kemptide insert, 100 pM oligonucleotides (sense: 5'-A GAA CTG CGC CGG GCC AGC CTG GGC-3'; reverse: 5'-A TCC GCC CAG GCT GGC CCG GCG CAG -3') were heated to 95°C and allowed to cool to room temperature. The resulting DNA was diluted 1:10 and phosphorylated according to manufacturer's protocol using T4-PNK kinase (Fermentas). After heat inactivating the kinase for 20 minutes at 60°C, DNA was ligated to Bbsl-opened mKCP-1 that had been dephosphorylated using shrimp alkaline phosphatase. This result was KCAP-1, a genetically encoded FRET-based probe carrying both a PKC and PKA phosphorylation sequence (Fig. 1).

KCAP-1 was cut using Ncol and BamHI restriction enzymes. The digest was separated by agarose gel electrophoresis, and the band containing pleckstrin and Kemptide coding regions was purified using a Qiagen gel extraction kit. Resulting DNA was co-ligated in frame with an EYPF and GFP2 cassette into a pET-9d N-His vector. After transforming E. coli cells, positive clones were confirmed by colony PCR and sequencing. Protein was then over-expressed in BL21 (DE3) at 25°C. Following ultrasonification, protein was purified on a Ni-chelate column under batch conditions. Buffer exchange was carried out using gel filtration columns.

N1E-115 cells and HeLa CCL-2 cells (LGC Promochem) were used for in vitro FRET experiments. Cells were cultured following manufacturer's recommendation. For microscopy, cells were seeded in 35 mm MatTek chambers and transfected overnight with Fugene (Roche) in Opti-MEM (Gibco) upon reaching 40-60% confluence. Two hours before microscopy, the Opti-MEM was exchanged for visualization medium (115 mM NaCl, 1.2 mM CaCl2, 1.2 mM MgCl₂, 2.4 mM K₂HPO₄, 20 mM HEPES, pH 7.4). Cells were stored at 37°C at all times, except during experiments performed with confocal microscopy. In this case, no heating chamber was available for the microscope and experiments were conducted at room temperature after dishes were equilibrated for 10 minutes.

Confocal microscopy was performed on a Leica SP2 AOBS microscope (Leica Microsystems). Excitation was carried out with a 20 mW 405 nm diode laser. EYFP and GFP² emission values were detected at 490-510 nm and 520-540 nm respectively.

Wide field microscopy was carried out on a Zeiss Axiovert microscope with a Visitron filter changing unit. An HBO lamp with 405/20 nm filter and 425 dclp dichroidic long pass mirror was used to excite samples at 405 nm. A CCD (charged coupled device) camera captured images after appropriate band pass filters were applied. Emission from GFP2 and EYFP were imaged using 505/40 and 535/30 band pass filters, respectively.

63X oil immersion objectives (Zeiss) were used for imaging and image analysis was carried out with Image J software (Rasband WS, Image J. National Institute of Health, Bethesda MD, USA http://rsb.info.nih.gov/ij/ , 1997-2004).

### 2. Generation of KAP-1 and KAP-2

KAP-1 and KAP-2 were created by replacing all phosphorylation-acceptor amino acids residues of pleckstrin's PKC phosphorylation site with alanine or glutamate residues, respectively. Briefly, KCAP-1 of Example 1 was digested using Xbal and gel purified. DNA fragments coding for the cloned Kemptide sequence were then ligated into an Xbal-opened version of the KCP-1 plasmid in which Ser-113, Thr-114, and Ser-117 had all been mutated to either alanine or glutamine residues. The alanine substitution renders the amino acid sequence unsusceptible to phosphorylation while the glutamine substitution mimics a constitutively phosphorylated form of the protein. Plasmids were verified by colony PCR and subsequent sequencing.

Expression and light microscopy were performed essentially as described for KCAP-1.

### 3. Determination of kinase activity

Various kinase activators and inhibitors used in this study are described in Table 1.

**Table 1: Activators and inhibitors used in this study**

| Activator/Inhibitor | Obtained from | Concentration | Solvent of Stock Solution |
|---|---|---|---|
| Bradykinin | Sigma-Aldrich | 1 µM | Water |
| 8-Br-cAMP/AM | Biolog | 50 µM | DMSO |
| TPA | CalBioChem | 200 nM | DMSO |
| Gö6983 | CalBioChem | 500 nM | DMSO |
| Forskolin | Sigma-Aldrich | 10 µM | DMSO |
| IBMX | Sigma-Aldrich | 100 µM | DMSO |
| Prostagiandin-E-1 (PGE1) | Sigma-Aldrich | 10 µM | DMSO/pluronic F-127 |

Fig. 3 shows that KCAP-1, KAP-1, and KAP-2 responded with a decrease in FRET efficiency when cells were treated with PKA activators like the physiological stimulus, prostaglandin E-1 and the non-degradable cAMP analog 8-Br-cAMP/AM. Only KCAP-1 gave a positive change in FRET efficiency following activation of PKC with TPA.

### 4. In vitro control of phosphorylation of KCAP-1

PKB, Ca²⁺ calmodulin dependent protein kinase II (CaMKII), and the catalytic subunit of PKA and were purchased from New England Biolabs. Phosphorylation assays using γ-³²P-ATP were conducted according to manufacturer's recommendations. Full-length PKCtheta with an N-terminal His-tag was purified from baculovirus-infected Sf9 cells. Phosphorylation was performed in the presence of 50mM Tris/Cl pH7.5, 10mM MgCl₂, 0.1 mM EGTA, 0.1 mM ATP, 0.2 mM DTT, 0.2 mg/ml phosphatidylserine, 0.02 mg/ml diacylglycerol, 0.03% Triton X-100. Phosphorylation assays with Aurora A and Aurora B contained 50 mM Tris/Cl pH 7.5, 50 mM NaCl, 1mM DTT, 10 mM MgCl2, 0.1 mM ATP. Reaction mixtures were separated by SDS-PAGE and visualized on Kodak X-Omat AR film (Fig. 4). For comparative purposes, KCP-1, a PKC probe lacking the Kemptide sequence is shown. KCAP-1 is phosphorylated by PKC-θ, PKA, Aurora A Kinase, Aurora B kinase and CamKll while KCP-1 appears to be sensitive to PKC alone.

### 5. Real-time in situ measurement

Cell images from N1E-115 cells transfected with KCAP-1 are shown in Fig. 5.

### 6. Generation of KCAP-1 ^{S244E} and KCAP-1^{S244A}

The same cloning procedure as for KCAP-1 was used to generate two constructs, KCAP-1 ^{S244E} and KCAP-1 ^{S244A}, with mutations to Ser244, the phosphoracceptor amino acid of KCAP-1's Kemptide sequence. Oligonucleotides used for this procedure are listed in Table 2. Plasmids were verified by sequencing.

**Table 2: KCAP-1 variants with mutations to the phosphoacceptor amino acid of the Kemptide sequence**

| Mutant Name | Sense Oligonucleotide** | Reverse Oligonucleotide** | Linker Amino Acid Sequence † |
|---|---|---|---|
| KCAP-1^{S244E} | 5'-AGAACTGCGCCGGG CCGAGCTGGGC-3' | 5'-ATCCGCCCAGCTCG GCCCGGCGCAG-3' | L R R A E L G |
| KCAP-1^{S244A} | 5'-AGAACTGCGCCG GGCCGCCCTGGGC-3' | 5'-ATCCGCCCAGGGC GGCCCGGCGCAG-3' | L R R A A L G |

| | | | |
|---|---|---|---|
| **Refers to oligonucleotides annealed for ligation with mKCP-1 † Linker added following amino acid 239 of KCP-1's pleckstrin coding region. Amino acids different from KCAP-1 appear in bold with underline. | | | |

Fig. 6 shows that mutations to Ser244, the putative phosphoacceptor amino acid of KCAP-1's Kemptide sequence, rendered the probe insensitive to PKA stimulation with 100 µM forskolin/10 µM IBMX. Subsequent stimulation with 200 nM TPA yields a positive FRET change like the unmodified KCP-1 probe. In particular, the alanine substitution renders the amino acid sequence unsusceptible to phosphorylation while the glutamine substitution mimics a constitutively phoshorylated form of the protein. Consequently, FRET changes following stimulation of PKA by forskolin were not observed. Subsequent stimulation with phorbol esters gave a normal PKC response relative to KCP-1.

### 7. Comparative example

Eighteen amino acids, comprising a loop located carboxyterminally to pleckstrin's DEP domain, were deleted from KCP-1 to create a separate PKC probe, KCP-2. A modified version of KCP-2, mKCP-2 (modified KCP-2), was created in a similar manner to mKCP-1, except that the reverse primer used in the first PCR step was 5'-TCA TGG ATC CCA GTC TTC GTA TTA GGA AGA CCT GTC TGG AAA GTA GTA-3'.

The impact of a particular mutation in KCP-1's carboxyterminal loop (amino acids 221-239 of the pleckstrin region) was studied by introducing oligonucleotides into mKCP-2 that coded for the carboxyterminal loop with a specific mutation. Mutants are listed in Table 3 and corresponding FRET behaviour is depicted in Fig. 2 A.

**Table 3: Mutations in KCP-1's Acidic Loop Region**

| Mutant Name | Sense Oligonucleotide* | Reverse Oligonucleotide* | Amino Acid Sequence of C-terminal loop† |
|---|---|---|---|
| KCP-1 | - | - | EENSSDD DVILKE |
| KCP-1^{E227Q} | 5'-AGACAGTGGGTTC TTCTGTCAGGAAAATT CCAGTGACGATGACGT GATTCTGAAAGAG-3' | 5'-ATCCCTCTTTCAGA ATCACGTCATCGTCAC TGGAATTTTCCTGAC AGAAGAACC CACT-3' | QENSSDD DVILKE |
| KCP-1^{E228Q} | 5'-AGACAGTGGGTTCT TCTGTGAGCAGAATTC CAGTGACGATGACGTG ATTCTGAAAGAG-3' | 5'-ATCCCTCTTTCAGAA TCACGTCATCGTCACTGGA ATTCTGCTCACAGAAGAACC CACT-3' | EQNSSDD D V I L K E |
| KCP-1^{EE227-8QQ} | 5'-AGACAGTGGGTTCT TCTGTCAACAGAATTC CAGTGACGATGACGT GATTCTGAAAGAG-3' | 5'-ATCCCTCTTTCAGA ATCACGTCATCGTCAC TGGAATTCTGTTGACA GAAGAACCCACT-3' | QQNSSDD D VILK E |
| KCP-1^{DDD232-} 4NNN | 5'-AGACAGTGGGTTCTT CTGTGAGGAAAATTCC AGTGACGATGACGTGA TTCTGCTGGAG-3' | 5'-ATCCCTCCAGCAGA ATCACGTCATCGTCACTGG AATTTTCCTCACAGA AGAACCCACT-3' | E E N S S N N N V I L K E |
| KCP-1^{E239Q} | 5'-AGACAGTGGGTTCTT CTGTGAGGAAAATTCC AGTGACGATGACGTGA TTCTGAAACAG-3' | 5'-ATCCCTGTTTCAGA ATCACGTCATCGTCACTGG AATTTTCCTCACAG AAGAACCCACT-3' | EENSSDD D V I L K Q |
| KCP-1^{EE239} 8QQ;DDD232- 4NNN;EZ39Q | 5'-AGACAGTGGGTTC TTCTGTCAGCAAAATT CCAGTAACAATAACGT GATTCTGAAACAA-3' | 5'-ATCCTTGTTTCAG AATCACGTTATTGTTA CTGGAATTTTGCTGAC AGAAGAACCCACT-3' | Q Q N S S N N N V I L K Q |

| | | | |
|---|---|---|---|
| * Refers to oligonucleotides annealed for ligation with mKCP-1. † Sequence of amino acids 227-239 of pleckstrin coding region. Altered amino acids appear in bold with underline. | | | |

Oligonucleotides coding for various linkers were cloned into mKCP-1 yielding probes based on KCP-1, but with longer stretches between the pleckstrin and GFP² domains. The sequences for two of these constructs, KCP-1A and KCP-1C appear in Table 4 and corresponding FRET behaviour is depicted in Fig. 2 B.

**Table 4: KCP-1 variants with linker insertions between the pleckstrin and GFP² domains**

| Mutant Name | Sense Oligonucleotide** | Reverse Oligonucleotide** | Linker Amino Acid Sequence† |
|---|---|---|---|
| **KCP-1A** | 5'-AGAAAGCGAGACCG TGCTGGAGGACGTG-3' | 5'-ATCCCACGTCCTCCA GCACGGTCTCGCT-3' | E S E T V L E D V G |
| **KCP-1C** | 5'-AGAAGGCGGAGGTA GCCTGGTGCCCCGC-3' | 5'-ATCCGCGGGGCACCA GGCTACCTCCGCC-3' | EGGGSLVPRG |

| | | | |
|---|---|---|---|
| **Refers to oligonucleotides annealed for ligation with mKCP-1. | | | |

Mutations to a C-terminal loop located between KCP-1's DEP and GFP2 domains (amino acids 221-239 of the pleckstrin coding region) give a variety of FRET responses depending upon the alteration. Constructs carrying mutations to acidic amino acids in KCP-1's C-terminal loop differ widely in FRET response. Addition of amino acids between KCP-1's pleckstrin region and GFP2 did not largely impact FRET changes relative to the original KCP-1 probe.

## Claims

1. Fusion protein comprising two fluorophores, a pleckstrin PH domain, a pleckstrin DEP domain and at least one substrate region for an enzyme performing a covalent modification, wherein the pleckstrin PH domain and the pleckstrin DEP domain are located between the fluorophores, and the substrate region is located between one of the two pleckstrin domains and the fluorophor on its side.

2. Fusion protein according to claim 1, wherein the covalent modification of the substrate region induces a conformational change causing a detectable change in FRET between the fluorophores.

3. Fusion protein according to claim 1 or 2, wherein the DEP domain is located on the carboxyterminal side of the PH domain.

4. Fusion protein according to any one of claims 1 to 3, wherein the substrate region is located between the DEP domain and the fluorophor on its side.

5. Fusion protein according to any one of claims 1 to 4, wherein the region between the DEP domain and the substrate region comprises a negatively charged motif.

6. Fusion protein according to any one of claims 1 to 5, wherein the substrate region is suitable for phosphorylation by protein kinase A.

7. Fusion protein according to any one of claims 1 to 6, wherein there is at least one additional substrate region located in between the PH and the DEP domain of the fusion protein.

8. Fusion protein according to claim 7, wherein the additional substrate region is suitable for phosphorylation by protein kinase C.

9. Fusion protein according to any one of claims 1 to 6, wherein there is no additional substrate region located in between the PH and the DEP domain of the fusion protein.

10. Fusion protein according to any of claims 1 to 9, wherein the fluorophores are those of green and yellow fluorescent protein (GFP and YFP).

11. Fusion protein according to claim 1, whose sequence is identical with or homologous to that encoded by SEQ ID NO:1.

12. Fusion protein according to claim 1, whose sequence is identical with or homologous to that encoded by SEQ ID NO:2.

13. Fusion protein according to claim 1, whose sequence is identical with or homologous to that encoded by SEQ ID NO:3.

14. Nucleic acid, comprising a sequence encoding the amino acid sequence of a fusion protein according to any one of claims 1 to 13.

15. Cell or cell line expressing at least the amino acid sequence of one fusion protein according to any one of claims 1 to 13.

16. Transgenic multicellular non-human organism expressing at least the amino acid sequence of a protein according to any one of claims 1 to 13.

17. Organism according to claim 16, wherein the expression is controlled by a tissue-specific, developmentally regulated or externally controlled promoter.

18. Method for determination of the activity of at least one enzyme performing covalent modifications of a polypeptide, wherein the method comprises the use of a fusion protein according to any of claims 1 to 13 and determination of FRET between the two fluorophores.

19. Method according to claim 18, wherein the fusion protein is as defined in claims 8 or 9 and the method is for the determination of the activities of at least two enzymes.

20. Method according to claim 18 or 19, wherein the use of the fusion protein comprises expressing the fusion protein in a cell.

21. Method according to any one of claims 18 to 20, wherein the enzyme whose activity is to be determined is protein kinase A and/or protein kinase C.

22. Method according to claim 21, wherein one of two enzymes whose activity is to be determined is protein kinase A and the other is protein kinase C.

23. Method according to any one of claims 18 to 22, wherein the method comprises the parallel use of n fusion proteins as defined in any one of claims 1 to 6 and 9, permitting the parallel determination of up to n enzyme activities.

24. Method according to any one of claims 14 to 18, wherein the method comprises the parallel use of n fusion proteins as defined in any one of claims 1 to 8, permitting the parallel determination of up to 2n enzyme activities.

25. Use of a fusion protein according to any one of claims 1 to 13 for determination of the activity of at least one enzyme performing covalent modifications of a polypeptide.

26. Kit for performing the method according to any one of claims 18 to 24, wherein the kit comprises a fusion protein according to any of claims 1 to 13 and/or a nucleic acid according to claim 14.

27. Kit according to claim 26 comprising at least one inhibitor for the selective blocking of a signalling pathway.

28. Kit according to claim 27, wherein the inhibitor is an inhibitor for PKC.


### Annex to the application documents - subsequently filed sequences listing

SEQUENCE LISTING

<110>  EMBL

<120>  Pleckstrin-based fusion protein and method for monitoring of enzyme
activities by FRET

<130>  M/46274

<140>  EP 05 021 808.0
<141>  2005-10-06

<160>  5

<170>  PatentIn version 3.1

<210>  1
<211>  6132
<212>  DNA
<213>  Artificial Sequence

<220>
<223>  KAP-1

<400>  1
tagttattaa tagtaatcaa ttacggggtc attagttcat agcccatata tggagttccg      60

cgttacataa cttacggtaa atggcccgcc tggctgaccg cccaacgacc cccgcccatt     120

gacgtcaata atgacgtatg ttcccatagt aacgccaata gggactttcc attgacgtca     180

atgggtggag tatttacggt aaactgccca cttggcagta catcaagtgt atcatatgcc     240

aagtacgccc cctattgacg tcaatgacgg taaatggccc gcctggcatt atgcccagta     300

catgacctta tgggactttc ctacttggca gtacatctac gtattagtca tcgctattac     360

catggtgatg cggttttggc agtacatcaa tgggcgtgga tagcggtttg actcacgggg     420

atttccaagt ctccacccca ttgacgtcaa tgggagtttg ttttggcacc aaaatcaacg     480

ggactttcca aaatgtcgta acaactccgc cccattgacg caaatgggcg gtaggcgtgt     540

acggtgggag gtctatataa gcagagctgg tttagtgaac cgtcagatcc gctagcgcta     600

ccggtcgcca ccatggtgag caagggcgag gagctgttca ccggggtggt gcccatcctg     660

gtcgagctgg acggcgacgt aaacggccac aagttcagcg tgtccggcga gggcgagggc     720

gatgccacct acggcaagct gaccctgaag ttcatctgca ccaccggcaa gctgcccgtg     780

ccctggccca ccctcgtgac caccttcggc tacggcctgc agtgcttcgc ccgctacccc     840

gaccacatga agcagcacga cttcttcaag tccgccatgc ccgaaggcta cgtccaggag     900

cgcaccatct tcttcaagga cgacggcaac tacaagaccc gcgccgaggt gaagttcgag     960

ggcgacaccc tggtgaaccg catcgagctg aagggcatcg acttcaagga ggacggcaac    1020

atcctggggc acaagctgga gtacaactac aacagccaca acgtctatat catggccgac    1080

aagcagaaga acggcatcaa ggtgaacttc aagatccgcc acaacatcga ggacggcagc    1140

gtgcagctcg ccgaccacta ccagcagaac acccccatcg gcgacggccc cgtgctgctg 1200
cccgacaacc actacctgag ctaccagtcc gccctgagca aagaccccaa cgagaagcgc 1260
gatcacatgg tcctgctgga gttcgtgacc gccgccggga tcactctcgg catggacgag 1320
ctgtacaagt ccggagccat ggaaccaaag cggatcagag agggctacct tgtgaagaag 1380
gggagcgtgt tcaatacgtg gaaacccatg tgggttgtat tgttagaaga tggaattgaa 1440
ttctataaga agaaaagtga caacagcccc aaaggaatga tcccgctgaa agggagcact 1500
ctgactagcc cttgtcaaga ctttggcaaa aggatgtttg tgtttaagat cactacgacc 1560
aaacagcagg accacttctt ccaggcagcc ttcctggagg agagagatgc ctgggttcgg 1620
gatatcaata aggccattaa atgcattgaa ggaggccaga aatttgccag gaaagctgct 1680
aggagggcta ttcgactgcc agaaaccatt gacttaggtg ccttatattt gtccatgaaa 1740
gacactgaaa aaggaataaa agaactgaat ctagagaagg acaagaagat ttttaatcac 1800
tgcttcacag gtaactgcgt cattgattgg ctggtatcca accagtctgt taggaatcgc 1860
caggaaggcc tcatgattgc ttcatcgctg ctcaatgagg ggtatctgca gcctgctgga 1920
gacatgtcca agagtgcagt ggatggaact gctgaaaacc ctttcctgga caaccctgat 1980
gccttctact actttccaga cagtgggttc ttctgtgaag agaattccag tgatgatgat 2040
gtgattctga aagaactgcg ccgggccagc ctgggcggat ccatggtgag caagggcgag 2100
gagctgttca ccggggtggt gcccatcctg gtcgagctgg acggcgacgt aaacggccac 2160
aagttcagcg tgtccggcga gggcgagggc gatgccacct acggcaagct gaccctgaag 2220
ttcatctgca ccaccggcaa gctgcccgtg ccctggccca ccctcgtgac caccctgagc 2280
tacggcgtgc agtgcttcag ccgctacccc gaccacatga agcagcacga cttcttcaag 2340
tccgccatgc ccgaaggcta cgtccaggag cgcaccatct tcttcaagga cgacggcaac 2400
tacaagaccc gcgccgaggt gaagttcgag ggcgacaccc tggtgaaccg catcgagctg 2460
aagggcatcg acttcaagga ggacggcaac atcctggggc acaagctgga gtacaactac 2520
aacagccaca acgtctatat catggccgac aagcagaaga acggcatcaa ggtgaacttc 2580
aagatccgcc acaacatcga ggacggcagc gtgcagctcg ccgaccacta ccagcagaac 2640
acccccatcg gcgacggccc cgtgctgctg cccgacaacc actacctgag cacccagtcc 2700
gccctgagca aagaccccaa cgagaagcgc gatcacatgg tcctgctgga gttcgtgacc 2760
gccgccggga tcactctcgg catggacgag ctgtacaagt aatctagata actgatcata 2820
atcagccata ccacatttgt agaggtttta cttgctttaa aaaacctccc acacctcccc 2880
ctgaacctga aacataaaat gaatgcaatt gttgttgtta acttgtttat tgcagcttat 2940
aatggttaca aataaagcaa tagcatcaca aatttcacaa ataaagcatt tttttcactg 3000
cattctagtt gtggtttgtc caaactcatc aatgtatctt aaggcgtaaa ttgtaagcgt 3060

taatattttg ttaaaattcg cgttaaattt ttgttaaatc agctcatttt ttaaccaata 3120

ggccgaaatc ggcaaaatcc cttataaatc aaaagaatag accgagatag ggttgagtgt 3180

tgttccagtt tggaacaaga gtccactatt aaagaacgtg gactccaacg tcaaagggcg 3240

aaaaaccgtc tatcagggcg atggcccact acgtgaacca tcaccctaat caagtttttt 3300

ggggtcgagg tgccgtaaag cactaaatcg gaaccctaaa gggagccccc gatttagagc 3360

ttgacgggga aagccggcga acgtggcgag aaaggaaggg aagaaagcga aaggagcggg 3420

cgctagggcg ctggcaagtg tagcggtcac gctgcgcgta accaccacac ccgccgcgct 3480

taatgcgccg ctacagggcg cgtcaggtgg cacttttcgg ggaaatgtgc gcggaacccc 3540

tatttgttta tttttctaaa tacattcaaa tatgtatccg ctcatgagac aataaccctg 3600

ataaatgctt caataatatt gaaaaaggaa gagtcctgag gcggaaagaa ccagctgtgg 3660

aatgtgtgtc agttagggtg tggaaagtcc ccaggctccc cagcaggcag aagtatgcaa 3720

agcatgcatc tcaattagtc agcaaccagg tgtggaaagt ccccaggctc cccagcaggc 3780

agaagtatgc aaagcatgca tctcaattag tcagcaacca tagtcccgcc cctaactccg 3840

cccatcccgc ccctaactcc gcccagttcc gcccattctc cgccccatgg ctgactaatt 3900

ttttttattt atgcagaggc cgaggccgcc tcggcctctg agctattcca gaagtagtga 3960

ggaggctttt ttggaggcct aggcttttgc aaagatcgat caagagacag gatgaggatc 4020

gtttcgcatg attgaacaag atggattgca cgcaggttct ccggccgctt gggtggagag 4080

gctattcggc tatgactggg cacaacagac aatcggctgc tctgatgccg ccgtgttccg 4140

gctgtcagcg caggggcgcc cggttctttt tgtcaagacc gacctgtccg gtgccctgaa 4200

tgaactgcaa gacgaggcag cgcggctatc gtggctggcc acgacgggcg ttccttgcgc 4260

agctgtgctc gacgttgtca ctgaagcggg aagggactgg ctgctattgg gcgaagtgcc 4320

ggggcaggat ctcctgtcat ctcaccttgc tcctgccgag aaagtatcca tcatggctga 4380

tgcaatgcgg cggctgcata cgcttgatcc ggctacctgc ccattcgacc accaagcgaa 4440

acatcgcatc gagcgagcac gtactcggat ggaagccggt cttgtcgatc aggatgatct 4500

ggacgaagag catcaggggc tcgcgccagc cgaactgttc gccaggctca aggcgagcat 4560

gcccgacggc gaggatctcg tcgtgaccca tggcgatgcc tgcttgccga atatcatggt 4620

ggaaaatggc cgcttttctg gattcatcga ctgtggccgg ctgggtgtgg cggaccgcta 4680

tcaggacata gcgttggcta cccgtgatat tgctgaagag cttggcggcg aatgggctga 4740

ccgcttcctc gtgctttacg gtatcgccgc tcccgattcg cagcgcatcg ccttctatcg 4800

ccttcttgac gagttcttct gagcgggact ctggggttcg aaatgaccga ccaagcgacg 4860

cccaacctgc catcacgaga tttcgattcc accgccgcct tctatgaaag gttgggcttc 4920

ggaatcgttt tccgggacgc cggctggatg atcctccagc gcggggatct catgctggag 4980

ttcttcgccc accctagggg gaggctaact gaaacacgga aggagacaat accggaagga 5040

acccgcgcta tgacggcaat aaaaagacag aataaaacgc acggtgttgg gtcgtttgtt 5100

cataaacgcg gggttcggtc ccagggctgg cactctgtcg ataccccacc gagaccccat 5160

tggggccaat acgcccgcgt ttcttccttt tccccacccc accccccaag ttcgggtgaa 5220

ggcccagggc tcgcagccaa cgtcggggcg gcaggccctg ccatagcctc aggttactca 5280

tatatacttt agattgattt aaaacttcat ttttaattta aaaggatcta ggtgaagatc 5340

ctttttgata atctcatgac caaaatccct taacgtgagt tttcgttcca ctgagcgtca 5400

gaccccgtag aaaagatcaa aggatcttct tgagatcctt tttttctgcg cgtaatctgc 5460

tgcttgcaaa caaaaaaacc accgctacca gcggtggttt gtttgccgga tcaagagcta 5520

ccaactcttt ttccgaaggt aactggcttc agcagagcgc agataccaaa tactgtcctt 5580

ctagtgtagc cgtagttagg ccaccacttc aagaactctg tagcaccgcc tacatacctc 5640

gctctgctaa tcctgttacc agtggctgct gccagtggcg ataagtcgtg tcttaccggg 5700

ttggactcaa gacgatagtt accggataag gcgcagcggt cgggctgaac ggggggttcg 5760

tgcacacagc ccagcttgga gcgaacgacc tacaccgaac tgagatacct acagcgtgag 5820

ctatgagaaa gcgccacgct tcccgaaggg agaaaggcgg acaggtatcc ggtaagcggc 5880

agggtcggaa caggagagcg cacgagggag cttccagggg gaaacgcctg gtatctttat 5940

agtcctgtcg ggtttcgcca cctctgactt gagcgtcgat ttttgtgatg ctcgtcaggg 6000

gggcggagcc tatggaaaaa cgccagcaac gcggcctttt tacggttcct ggccttttgc 6060

tggccttttg ctcacatgtt ctttcctgcg ttatcccctg attctgtgga taaccgtatt 6120

accgccatgc at 6132

<210> 2
<211> 6132
<212> DNA
<213> Artificial Sequence

<220>
<223> KAP-2

<400> 2
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggtcgcca ccatggtgag caagggcgag gagctgttca ccggggtggt gcccatcctg 660

gtcgagctgg acggcgacgt aaacggccac aagttcagcg tgtccggcga gggcgagggc 720

gatgccacct acggcaagct gaccctgaag ttcatctgca ccaccggcaa gctgcccgtg 780

ccctggccca ccctcgtgac caccttcggc tacggcctgc agtgcttcgc ccgctacccc 840

gaccacatga agcagcacga cttcttcaag tccgccatgc ccgaaggcta cgtccaggag 900

cgcaccatct tcttcaagga cgacggcaac tacaagaccc gcgccgaggt gaagttcgag 960

ggcgacaccc tggtgaaccg catcgagctg aagggcatcg acttcaagga ggacggcaac 1020

atcctggggc acaagctgga gtacaactac aacagccaca acgtctatat catggccgac 1080

aagcagaaga acggcatcaa ggtgaacttc aagatccgcc acaacatcga ggacggcagc 1140

gtgcagctcg ccgaccacta ccagcagaac acccccatcg gcgacggccc cgtgctgctg 1200

cccgacaacc actacctgag ctaccagtcc gccctgagca aagaccccaa cgagaagcgc 1260

gatcacatgg tcctgctgga gttcgtgacc gccgccggga tcactctcgg catggacgag 1320

ctgtacaagt ccggagccat ggaaccaaag cggatcagag agggctacct tgtgaagaag 1380

gggagcgtgt tcaatacgtg gaaacccatg tgggttgtat tgttagaaga tggaattgaa 1440

ttctataaga agaaaagtga caacagcccc aaaggaatga tcccgctgaa agggagcact 1500

ctgactagcc cttgtcaaga ctttggcaaa aggatgtttg tgtttaagat cactacgacc 1560

aaacagcagg accacttctt ccaggcagcc ttcctggagg agagagatgc ctgggttcgg 1620

gatatcaata aggccattaa atgcattgaa ggaggccaga aatttgccag gaaagaagaa 1680

aggagggaaa ttcgactgcc agaaaccatt gacttaggtg ccttatattt gtccatgaaa 1740

gacactgaaa aaggaataaa agaactgaat ctagagaagg acaagaagat ttttaatcac 1800

tgcttcacag gtaactgcgt cattgattgg ctggtatcca accagtctgt taggaatcgc 1860

caggaaggcc tcatgattgc ttcatcgctg ctcaatgagg ggtatctgca gcctgctgga 1920

gacatgtcca agagtgcagt ggatggaact gctgaaaacc ctttcctgga caaccctgat 1980

gccttctact actttccaga cagtgggttc ttctgtgaag agaattccag tgatgatgat 2040

gtgattctga aagaactgcg ccgggccagc ctgggcggat ccatggtgag caagggcgag 2100

gagctgttca ccggggtggt gcccatcctg gtcgagctgg acggcgacgt aaacggccac 2160

aagttcagcg tgtccggcga gggcgagggc gatgccacct acggcaagct gaccctgaag 2220

ttcatctgca ccaccggcaa gctgcccgtg ccctggccca ccctcgtgac caccctgagc 2280

tacggcgtgc agtgcttcag ccgctacccc gaccacatga agcagcacga cttcttcaag 2340

tccgccatgc ccgaaggcta cgtccaggag cgcaccatct tcttcaagga cgacggcaac 2400

tacaagaccc gcgccgaggt gaagttcgag ggcgacaccc tggtgaaccg catcgagctg 2460

aagggcatcg acttcaagga ggacggcaac atcctggggc acaagctgga gtacaactac 2520

aacagccaca acgtctatat catggccgac aagcagaaga acggcatcaa ggtgaacttc 2580

aagatccgcc acaacatcga ggacggcagc gtgcagctcg ccgaccacta ccagcagaac 2640

acccccatcg gcgacggccc cgtgctgctg cccgacaacc actacctgag cacccagtcc 2700

gccctgagca aagaccccaa cgagaagcgc gatcacatgg tcctgctgga gttcgtgacc 2760

gccgccggga tcactctcgg catggacgag ctgtacaagt aatctagata actgatcata 2820

atcagccata ccacatttgt agaggtttta cttgctttaa aaaacctccc acacctcccc 2880

ctgaacctga aacataaaat gaatgcaatt gttgttgtta acttgtttat tgcagcttat 2940

aatggttaca aataaagcaa tagcatcaca aatttcacaa ataaagcatt tttttcactg 3000

cattctagtt gtggtttgtc caaactcatc aatgtatctt aaggcgtaaa ttgtaagcgt 3060

taatattttg ttaaaattcg cgttaaattt ttgttaaatc agctcatttt ttaaccaata 3120

ggccgaaatc ggcaaaatcc cttataaatc aaaagaatag accgagatag ggttgagtgt 3180

tgttccagtt tggaacaaga gtccactatt aaagaacgtg gactccaacg tcaaagggcg 3240

aaaaaccgtc tatcagggcg atggcccact acgtgaacca tcaccctaat caagtttttt 3300

ggggtcgagg tgccgtaaag cactaaatcg gaaccctaaa gggagccccc gatttagagc 3360

ttgacgggga aagccggcga acgtggcgag aaaggaaggg aagaaagcga aaggagcggg 3420

cgctagggcg ctggcaagtg tagcggtcac gctgcgcgta accaccacac ccgccgcgct 3480

taatgcgccg ctacagggcg cgtcaggtgg cacttttcgg ggaaatgtgc gcggaacccc 3540

tatttgttta tttttctaaa tacattcaaa tatgtatccg ctcatgagac aataaccctg 3600

ataaatgctt caataatatt gaaaaaggaa gagtcctgag gcggaaagaa ccagctgtgg 3660

aatgtgtgtc agttagggtg tggaaagtcc ccaggctccc cagcaggcag aagtatgcaa 3720

agcatgcatc tcaattagtc agcaaccagg tgtggaaagt ccccaggctc cccagcaggc 3780

agaagtatgc aaagcatgca tctcaattag tcagcaacca tagtcccgcc cctaactccg 3840

cccatcccgc ccctaactcc gcccagttcc gcccattctc cgccccatgg ctgactaatt 3900

ttttttattt atgcagaggc cgaggccgcc tcggcctctg agctattcca gaagtagtga 3960

ggaggctttt ttggaggcct aggcttttgc aaagatcgat caagagacag gatgaggatc 4020

gtttcgcatg attgaacaag atggattgca cgcaggttct ccggccgctt gggtggagag 4080

gctattcggc tatgactggg cacaacagac aatcggctgc tctgatgccg ccgtgttccg 4140

gctgtcagcg caggggcgcc cggttctttt tgtcaagacc gacctgtccg gtgccctgaa 4200
tgaactgcaa gacgaggcag cgcggctatc gtggctggcc acgacgggcg ttccttgcgc 4260
agctgtgctc gacgttgtca ctgaagcggg aagggactgg ctgctattgg gcgaagtgcc 4320
ggggcaggat ctcctgtcat ctcaccttgc tcctgccgag aaagtatcca tcatggctga 4380
tgcaatgcgg cggctgcata cgcttgatcc ggctacctgc ccattcgacc accaagcgaa 4440
acatcgcatc gagcgagcac gtactcggat ggaagccggt cttgtcgatc aggatgatct 4500
ggacgaagag catcaggggc tcgcgccagc cgaactgttc gccaggctca aggcgagcat 4560
gcccgacggc gaggatctcg tcgtgaccca tggcgatgcc tgcttgccga atatcatggt 4620
ggaaaatggc cgcttttctg gattcatcga ctgtggccgg ctgggtgtgg cggaccgcta 4680
tcaggacata gcgttggcta cccgtgatat tgctgaagag cttggcggcg aatgggctga 4740
ccgcttcctc gtgctttacg gtatcgccgc tcccgattcg cagcgcatcg ccttctatcg 4800
ccttcttgac gagttcttct gagcgggact ctggggttcg aaatgaccga ccaagcgacg 4860
cccaacctgc catcacgaga tttcgattcc accgccgcct tctatgaaag gttgggcttc 4920
ggaatcgttt tccgggacgc cggctggatg atcctccagc gcggggatct catgctggag 4980
ttcttcgccc accctagggg gaggctaact gaaacacgga aggagacaat accggaagga 5040
acccgcgcta tgacggcaat aaaaagacag aataaaacgc acggtgttgg gtcgtttgtt 5100
cataaacgcg gggttcggtc ccagggctgg cactctgtcg ataccccacc gagaccccat 5160
tggggccaat acgcccgcgt ttcttccttt tccccacccc accccccaag ttcgggtgaa 5220
ggcccagggc tcgcagccaa cgtcggggcg gcaggccctg ccatagcctc aggttactca 5280
tatatacttt agattgattt aaaacttcat ttttaattta aaaggatcta ggtgaagatc 5340
ctttttgata atctcatgac caaaatccct taacgtgagt tttcgttcca ctgagcgtca 5400
gaccccgtag aaaagatcaa aggatcttct tgagatcctt tttttctgcg cgtaatctgc 5460
tgcttgcaaa caaaaaaacc accgctacca gcggtggttt gtttgccgga tcaagagcta 5520
ccaactcttt ttccgaaggt aactggcttc agcagagcgc agataccaaa tactgtcctt 5580
ctagtgtagc cgtagttagg ccaccacttc aagaactctg tagcaccgcc tacatacctc 5640
gctctgctaa tcctgttacc agtggctgct gccagtggcg ataagtcgtg tcttaccggg 5700
ttggactcaa gacgatagtt accggataag gcgcagcggt cgggctgaac ggggggttcg 5760
tgcacacagc ccagcttgga gcgaacgacc tacaccgaac tgagatacct acagcgtgag 5820
ctatgagaaa gcgccacgct tcccgaaggg agaaaggcgg acaggtatcc ggtaagcggc 5880
agggtcggaa caggagagcg cacgagggag cttccagggg gaaacgcctg gtatctttat 5940
agtcctgtcg ggtttcgcca cctctgactt gagcgtcgat ttttgtgatg ctcgtcaggg 6000

gggcggagcc tatggaaaaa cgccagcaac gcggcctttt tacggttcct ggccttttgc 6060

tggccttttg ctcacatgtt ctttcctgcg ttatcccctg attctgtgga taaccgtatt 6120

accgccatgc at 6132

<210> 3
<211> 6132
<212> DNA
<213> Artificial Sequence

<220>
<223> KACP-1

<400> 3
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggtcgcca ccatggtgag caagggcgag gagctgttca ccggggtggt gcccatcctg 660

gtcgagctgg acggcgacgt aaacggccac aagttcagcg tgtccggcga gggcgagggc 720

gatgccacct acggcaagct gaccctgaag ttcatctgca ccaccggcaa gctgcccgtg 780

ccctggccca ccctcgtgac caccttcggc tacggcctgc agtgcttcgc ccgctacccc 840

gaccacatga agcagcacga cttcttcaag tccgccatgc ccgaaggcta cgtccaggag 900

cgcaccatct tcttcaagga cgacggcaac tacaagaccc gcgccgaggt gaagttcgag 960

ggcgacaccc tggtgaaccg catcgagctg aagggcatcg acttcaagga ggacggcaac 1020

atcctggggc acaagctgga gtacaactac aacagccaca acgtctatat catggccgac 1080

aagcagaaga acggcatcaa ggtgaacttc aagatccgcc acaacatcga ggacggcagc 1140

gtgcagctcg ccgaccacta ccagcagaac acccccatcg gcgacggccc cgtgctgctg 1200

cccgacaacc actacctgag ctaccagtcc gccctgagca aagaccccaa cgagaagcgc 1260

gatcacatgg tcctgctgga gttcgtgacc gccgccggga tcactctcgg catggacgag 1320

ctgtacaagt ccggagccat ggaaccaaag cggatcagag agggctacct tgtgaagaag 1380

gggagcgtgt tcaatacgtg gaaacccatg tgggttgtat tgttagaaga tggaattgaa 1440

ttctataaga agaaaagtga caacagcccc aaaggaatga tcccgctgaa agggagcact 1500

ctgactagcc cttgtcaaga ctttggcaaa aggatgtttg tgtttaagat cactacgacc 1560

aaacagcagg accacttctt ccaggcagcc ttcctggagg agagagatgc ctgggttcgg 1620

gatatcaata aggccattaa atgcattgaa ggaggccaga aatttgccag gaaatctacc 1680

aggaggtcca ttcgactgcc agaaaccatt gacttaggtg ccttatattt gtccatgaaa 1740

gacactgaaa aaggaataaa agaactgaat ctagagaagg acaagaagat ttttaatcac 1800

tgcttcacag gtaactgcgt cattgattgg ctggtatcca accagtctgt taggaatcgc 1860

caggaaggcc tcatgattgc ttcatcgctg ctcaatgagg ggtatctgca gcctgctgga 1920

gacatgtcca agagtgcagt ggatggaact gctgaaaacc ctttcctgga caaccctgat 1980

gccttctact actttccaga cagtgggttc ttctgtgaag agaattccag tgatgatgat 2040

gtgattctga aagaactgcg ccgggccagc ctggcggatc ccatggtgag caagggcgag 2100

gagctgttca ccggggtggt gcccatcctg gtcgagctgg acggcgacgt aaacggccac 2160

aagttcagcg tgtccggcga gggcgagggc gatgccacct acggcaagct gaccctgaag 2220

ttcatctgca ccaccggcaa gctgcccgtg ccctggccca ccctcgtgac caccctgagc 2280

tacggcgtgc agtgcttcag ccgctacccc gaccacatga agcagcacga cttcttcaag 2340

tccgccatgc ccgaaggcta cgtccaggag cgcaccatct tcttcaagga cgacggcaac 2400

tacaagaccc gcgccgaggt gaagttcgag ggcgacaccc tggtgaaccg catcgagctg 2460

aagggcatcg acttcaagga ggacggcaac atcctggggc acaagctgga gtacaactac 2520

aacagccaca acgtctatat catggccgac aagcagaaga acggcatcaa ggtgaacttc 2580

aagatccgcc acaacatcga ggacggcagc gtgcagctcg ccgaccacta ccagcagaac 2640

acccccatcg gcgacggccc cgtgctgctg cccgacaacc actacctgag cacccagtcc 2700

gccctgagca aagaccccaa cgagaagcgc gatcacatgg tcctgctgga gttcgtgacc 2760

gccgccggga tcactctcgg catggacgag ctgtacaagt aatctagata actgatcata 2820

atcagccata ccacatttgt agaggtttta cttgctttaa aaaacctccc acacctcccc 2880

ctgaacctga aacataaaat gaatgcaatt gttgttgtta acttgtttat tgcagcttat 2940

aatggttaca aataaagcaa tagcatcaca aatttcacaa ataaagcatt tttttcactg 3000

cattctagtt gtggtttgtc caaactcatc aatgtatctt aaggcgtaaa ttgtaagcgt 3060

taatattttg ttaaaattcg cgttaaattt ttgttaaatc agctcatttt ttaaccaata 3120

ggccgaaatc ggcaaaatcc cttataaatc aaaagaatag accgagatag ggttgagtgt 3180

tgttccagtt tggaacaaga gtccactatt aaagaacgtg gactccaacg tcaaagggcg 3240

aaaaaccgtc tatcagggcg atggcccact acgtgaacca tcaccctaat caagtttttt 3300

ggggtcgagg tgccgtaaag cactaaatcg gaaccctaaa gggagccccc gatttagagc 3360

ttgacgggga aagccggcga acgtggcgag aaaggaaggg aagaaagcga aaggagcggg 3420

cgctagggcg ctggcaagtg tagcggtcac gctgcgcgta accaccacac ccgccgcgct 3480

taatgcgccg ctacagggcg cgtcaggtgg cacttttcgg ggaaatgtgc gcggaacccc 3540

tatttgttta tttttctaaa tacattcaaa tatgtatccg ctcatgagac aataaccctg 3600

ataaatgctt caataatatt gaaaaaggaa gagtcctgag gcggaaagaa ccagctgtgg 3660

aatgtgtgtc agttagggtg tggaaagtcc ccaggctccc cagcaggcag aagtatgcaa 3720

agcatgcatc tcaattagtc agcaaccagg tgtggaaagt ccccaggctc cccagcaggc 3780

agaagtatgc aaagcatgca tctcaattag tcagcaacca tagtcccgcc cctaactccg 3840

cccatcccgc ccctaactcc gcccagttcc gcccattctc cgccccatgg ctgactaatt 3900

ttttttattt atgcagaggc cgaggccgcc tcggcctctg agctattcca gaagtagtga 3960

ggaggctttt ttggaggcct aggcttttgc aaagatcgat caagagacag gatgaggatc 4020

gtttcgcatg attgaacaag atggattgca cgcaggttct ccggccgctt gggtggagag 4080

gctattcggc tatgactggg cacaacagac aatcggctgc tctgatgccg ccgtgttccg 4140

gctgtcagcg caggggcgcc cggttctttt tgtcaagacc gacctgtccg gtgccctgaa 4200

tgaactgcaa gacgaggcag cgcggctatc gtggctggcc acgacgggcg ttccttgcgc 4260

agctgtgctc gacgttgtca ctgaagcggg aagggactgg ctgctattgg gcgaagtgcc 4320

ggggcaggat ctcctgtcat ctcaccttgc tcctgccgag aaagtatcca tcatggctga 4380

tgcaatgcgg cggctgcata cgcttgatcc ggctacctgc ccattcgacc accaagcgaa 4440

acatcgcatc gagcgagcac gtactcggat ggaagccggt cttgtcgatc aggatgatct 4500

ggacgaagag catcaggggc tcgcgccagc cgaactgttc gccaggctca aggcgagcat 4560

gcccgacggc gaggatctcg tcgtgaccca tggcgatgcc tgcttgccga atatcatggt 4620

ggaaaatggc cgcttttctg gattcatcga ctgtggccgg ctgggtgtgg cggaccgcta 4680

tcaggacata gcgttggcta cccgtgatat tgctgaagag cttggcggcg aatgggctga 4740

ccgcttcctc gtgctttacg gtatcgccgc tcccgattcg cagcgcatcg ccttctatcg 4800

ccttcttgac gagttcttct gagcgggact ctggggttcg aaatgaccga ccaagcgacg 4860

cccaacctgc catcacgaga tttcgattcc accgccgcct tctatgaaag gttgggcttc 4920

ggaatcgttt tccgggacgc cggctggatg atcctccagc gcggggatct catgctggag 4980

ttcttcgccc accctagggg gaggctaact gaaacacgga aggagacaat accggaagga 5040

acccgcgcta tgacggcaat aaaaagacag aataaaacgc acggtgttgg gtcgtttgtt 5100

cataaacgcg gggttcggtc ccagggctgg cactctgtcg ataccccacc gagaccccat 5160

tggggccaat acgcccgcgt ttcttccttt tccccacccc accccccaag ttcgggtgaa 5220

ggcccagggc tcgcagccaa cgtcggggcg gcaggccctg ccatagcctc aggttactca 5280

tatatacttt agattgattt aaaacttcat ttttaattta aaaggatcta ggtgaagatc 5340

ctttttgata atctcatgac caaaatccct taacgtgagt tttcgttcca ctgagcgtca 5400

gaccccgtag aaaagatcaa aggatcttct tgagatcctt tttttctgcg cgtaatctgc 5460

tgcttgcaaa caaaaaaacc accgctacca gcggtggttt gtttgccgga tcaagagcta 5520

ccaactcttt ttccgaaggt aactggcttc agcagagcgc agataccaaa tactgtcctt 5580

ctagtgtagc cgtagttagg ccaccacttc aagaactctg tagcaccgcc tacatacctc 5640

gctctgctaa tcctgttacc agtggctgct gccagtggcg ataagtcgtg tcttaccggg 5700

ttggactcaa gacgatagtt accggataag gcgcagcggt cgggctgaac ggggggttcg 5760

tgcacacagc ccagcttgga gcgaacgacc tacaccgaac tgagatacct acagcgtgag 5820

ctatgagaaa gcgccacgct tcccgaaggg agaaaggcgg acaggtatcc ggtaagcggc 5880

agggtcggaa caggagagcg cacgagggag cttccagggg gaaacgcctg gtatctttat 5940

agtcctgtcg ggtttcgcca cctctgactt gagcgtcgat ttttgtgatg ctcgtcaggg 6000

gggcggagcc tatggaaaaa cgccagcaac gcggcctttt tacggttcct ggccttttgc 6060

tggccttttg ctcacatgtt ctttcctgcg ttatcccctg attctgtgga taaccgtatt 6120

accgccatgc at 6132

<210> 4
<211> 717
<212> DNA
<213> Artificial Sequence

<220>
<223> GFP2

<400> 4
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgagcta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaag 717

<210> 5
<211> 717
<212> DNA
<213> Artificial Sequence

<220>
<223> EYFP

<400> 5
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccttcggcta cggcctgcag tgcttcgccc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagct accagtccgc cctgagcaaa gaccccaacg agaagcgcga tcacatggtc 660

ctgctggagt tcgtgaccgc cgccgggatc actctcggca tggacgagct gtacaag 717

SEQUENCE LISTING

<110> EMBL

<120> Pleckstrin-based fusion protein and method for monitoring of enzyme
activities by FRET

<130> M/46274

<160> 5

<170> PatentIn version 3.1

<210> 1
<211> 6132
<212> DNA
<213> Artificial

<400> 1
tagttattaa tagtaatcaa ttacggggtc attagttcat agcccatata tggagttccg 60
cgttacataa cttacggtaa atggcccgcc tggctgaccg cccaacgacc cccgcccatt 120
gacgtcaata atgacgtatg ttcccatagt aacgccaata gggactttcc attgacgtca 180
atgggtggag tatttacggt aaactgccca cttggcagta catcaagtgt atcatatgcc 240
aagtacgccc cctattgacg tcaatgacgg taaatggccc gcctggcatt atgcccagta 300
catgacctta tgggactttc ctacttggca gtacatctac gtattagtca tcgctattac 360
catggtgatg cggttttggc agtacatcaa tgggcgtgga tagcggtttg actcacgggg 420
atttccaagt ctccacccca ttgacgtcaa tgggagtttg ttttggcacc aaaatcaacg 480
ggactttcca aaatgtcgta acaactccgc cccattgacg caaatgggcg gtaggcgtgt 540
acggtgggag gtctatataa gcagagctgg tttagtgaac cgtcagatcc gctagcgcta 600
ccggtcgcca ccatggtgag caagggcgag gagctgttca ccggggtggt gcccatcctg 660
gtcgagctgg acggcgacgt aaacggccac aagttcagcg tgtccggcga gggcgagggc 720
gatgccacct acggcaagct gaccctgaag ttcatctgca ccaccggcaa gctgcccgtg 780
ccctggccca ccctcgtgac caccttcggc tacggcctgc agtgcttcgc ccgctacccc 840

gaccacatga agcagcacga cttcttcaag tccgccatgc ccgaaggcta cgtccaggag 900
cgcaccatct tcttcaagga cgacggcaac tacaagaccc gcgccgaggt gaagttcgag 960
ggcgacaccc tggtgaaccg catcgagctg aagggcatcg acttcaagga ggacggcaac 1020
atcctggggc acaagctgga gtacaactac aacagccaca acgtctatat catggccgac 1080
aagcagaaga acggcatcaa ggtgaacttc aagatccgcc acaacatcga ggacggcagc 1140
gtgcagctcg ccgaccacta ccagcagaac acccccatcg gcgacggccc cgtgctgctg 1200
cccgacaacc actacctgag ctaccagtcc gccctgagca aagaccccaa cgagaagcgc 1260
gatcacatgg tcctgctgga gttcgtgacc gccgccggga tcactctcgg catggacgag 1320
ctgtacaagt ccggagccat ggaaccaaag cggatcagag agggctacct tgtgaagaag 1380
gggagcgtgt tcaatacgtg gaaacccatg tgggttgtat tgttagaaga tggaattgaa 1440
ttctataaga agaaaagtga caacagcccc aaaggaatga tcccgctgaa agggagcact 1500
ctgactagcc cttgtcaaga ctttggcaaa aggatgtttg tgtttaagat cactacgacc 1560
aaacagcagg accacttctt ccaggcagcc ttcctggagg agagagatgc ctgggttcgg 1620
gatatcaata aggccattaa atgcattgaa ggaggccaga aatttgccag gaaagctgct 1680
aggagggcta ttcgactgcc agaaaccatt gacttaggtg ccttatattt gtccatgaaa 1740
gacactgaaa aaggaataaa agaactgaat ctagagaagg acaagaagat ttttaatcac 1800
tgcttcacag gtaactgcgt cattgattgg ctggtatcca accagtctgt taggaatcgc 1860
caggaaggcc tcatgattgc ttcatcgctg ctcaatgagg ggtatctgca gcctgctgga 1920
gacatgtcca agagtgcagt ggatggaact gctgaaaacc ctttcctgga caaccctgat 1980
gccttctact actttccaga cagtgggttc ttctgtgaag agaattccag tgatgatgat 2040
gtgattctga aagaactgcg ccgggccagc ctgggcggat ccatggtgag caagggcgag 2100
gagctgttca ccggggtggt gcccatcctg gtcgagctgg acggcgacgt aaacggccac 2160
aagttcagcg tgtccggcga gggcgagggc gatgccacct acggcaagct gaccctgaag 2220
ttcatctgca ccaccggcaa gctgcccgtg ccctggccca ccctcgtgac caccctgagc 2280
tacggcgtgc agtgcttcag ccgctacccc gaccacatga agcagcacga cttcttcaag 2340
tccgccatgc ccgaaggcta cgtccaggag cgcaccatct tcttcaagga cgacggcaac 2400
tacaagaccc gcgccgaggt gaagttcgag ggcgacaccc tggtgaaccg catcgagctg 2460
aagggcatcg acttcaagga ggacggcaac atcctggggc acaagctgga gtacaactac 2520
aacagccaca acgtctatat catggccgac aagcagaaga acggcatcaa ggtgaacttc 2580
aagatccgcc acaacatcga ggacggcagc gtgcagctcg ccgaccacta ccagcagaac 2640
acccccatcg gcgacggccc cgtgctgctg cccgacaacc actacctgag cacccagtcc 2700

gccctgagca aagaccccaa cgagaagcgc gatcacatgg tcctgctgga gttcgtgacc 2760

gccgccggga tcactctcgg catggacgag ctgtacaagt aatctagata actgatcata 2820

atcagccata ccacatttgt agaggtttta cttgctttaa aaaacctccc acacctcccc 2880

ctgaacctga aacataaaat gaatgcaatt gttgttgtta acttgtttat tgcagcttat 2940

aatggttaca aataaagcaa tagcatcaca aatttcacaa ataaagcatt tttttcactg 3000

cattctagtt gtggtttgtc caaactcatc aatgtatctt aaggcgtaaa ttgtaagcgt 3060

taatattttg ttaaaattcg cgttaaattt ttgttaaatc agctcatttt ttaaccaata 3120

ggccgaaatc ggcaaaatcc cttataaatc aaaagaatag accgagatag ggttgagtgt 3180

tgttccagtt tggaacaaga gtccactatt aaagaacgtg gactccaacg tcaaagggcg 3240

aaaaaccgtc tatcagggcg atggcccact acgtgaacca tcaccctaat caagtttttt 3300

ggggtcgagg tgccgtaaag cactaaatcg gaaccctaaa gggagccccc gatttagagc 3360

ttgacgggga aagccggcga acgtggcgag aaaggaaggg aagaaagcga aaggagcggg 3420

cgctagggcg ctggcaagtg tagcggtcac gctgcgcgta accaccacac ccgccgcgct 3480

taatgcgccg ctacagggcg cgtcaggtgg cacttttcgg ggaaatgtgc gcggaacccc 3540

tatttgttta tttttctaaa tacattcaaa tatgtatccg ctcatgagac aataaccctg 3600

ataaatgctt caataatatt gaaaaaggaa gagtcctgag gcggaaagaa ccagctgtgg 3660

aatgtgtgtc agttagggtg tggaaagtcc ccaggctccc cagcaggcag aagtatgcaa 3720

agcatgcatc tcaattagtc agcaaccagg tgtggaaagt ccccaggctc cccagcaggc 3780

agaagtatgc aaagcatgca tctcaattag tcagcaacca tagtcccgcc cctaactccg 3840

cccatcccgc ccctaactcc gcccagttcc gcccattctc cgccccatgg ctgactaatt 3900

ttttttattt atgcagaggc cgaggccgcc tcggcctctg agctattcca gaagtagtga 3960

ggaggctttt ttggaggcct aggcttttgc aaagatcgat caagagacag gatgaggatc 4020

gtttcgcatg attgaacaag atggattgca cgcaggttct ccggccgctt gggtggagag 4080

gctattcggc tatgactggg cacaacagac aatcggctgc tctgatgccg ccgtgttccg 4140

gctgtcagcg caggggcgcc cggttctttt tgtcaagacc gacctgtccg gtgccctgaa 4200

tgaactgcaa gacgaggcag cgcggctatc gtggctggcc acgacgggcg ttccttgcgc 4260

agctgtgctc gacgttgtca ctgaagcggg aagggactgg ctgctattgg gcgaagtgcc 4320

ggggcaggat ctcctgtcat ctcaccttgc tcctgccgag aaagtatcca tcatggctga 4380

tgcaatgcgg cggctgcata cgcttgatcc ggctacctgc ccattcgacc accaagcgaa 4440

acatcgcatc gagcgagcac gtactcggat ggaagccggt cttgtcgatc aggatgatct 4500

ggacgaagag catcaggggc tcgcgccagc cgaactgttc gccaggctca aggcgagcat 4560

gcccgacggc gaggatctcg tcgtgaccca tggcgatgcc tgcttgccga atatcatggt 4620

ggaaaatggc cgcttttctg gattcatcga ctgtggccgg ctgggtgtgg cggaccgcta 4680

tcaggacata gcgttggcta cccgtgatat tgctgaagag cttggcggcg aatgggctga 4740

ccgcttcctc gtgctttacg gtatcgccgc tcccgattcg cagcgcatcg ccttctatcg 4800

ccttcttgac gagttcttct gagcgggact ctggggttcg aaatgaccga ccaagcgacg 4860

cccaacctgc catcacgaga tttcgattcc accgccgcct tctatgaaag gttgggcttc 4920

ggaatcgttt tccgggacgc cggctggatg atcctccagc gcggggatct catgctggag 4980

ttcttcgccc accctagggg gaggctaact gaaacacgga aggagacaat accggaagga 5040

acccgcgcta tgacggcaat aaaaagacag aataaaacgc acggtgttgg gtcgtttgtt 5100

cataaacgcg gggttcggtc ccagggctgg cactctgtcg ataccccacc gagaccccat 5160

tggggccaat acgcccgcgt ttcttccttt tccccacccc accccccaag ttcgggtgaa 5220

ggcccagggc tcgcagccaa cgtcggggcg gcaggccctg ccatagcctc aggttactca 5280

tatatacttt agattgattt aaaacttcat ttttaattta aaaggatcta ggtgaagatc 5340

ctttttgata atctcatgac caaaatccct taacgtgagt tttcgttcca ctgagcgtca 5400

gaccccgtag aaaagatcaa aggatcttct tgagatcctt tttttctgcg cgtaatctgc 5460

tgcttgcaaa caaaaaaacc accgctacca gcggtggttt gtttgccgga tcaagagcta 5520

ccaactcttt ttccgaaggt aactggcttc agcagagcgc agataccaaa tactgtcctt 5580

ctagtgtagc cgtagttagg ccaccacttc aagaactctg tagcaccgcc tacatacctc 5640

gctctgctaa tcctgttacc agtggctgct gccagtggcg ataagtcgtg tcttaccggg 5700

ttggactcaa gacgatagtt accggataag gcgcagcggt cgggctgaac ggggggttcg 5760

tgcacacagc ccagcttgga gcgaacgacc tacaccgaac tgagatacct acagcgtgag 5820

ctatgagaaa gcgccacgct tcccgaaggg agaaaggcgg acaggtatcc ggtaagcggc 5880

agggtcggaa caggagagcg cacgagggag cttccagggg gaaacgcctg gtatctttat 5940

agtcctgtcg ggtttcgcca cctctgactt gagcgtcgat ttttgtgatg ctcgtcaggg 6000

gggcggagcc tatggaaaaa cgccagcaac gcggcctttt tacggttcct ggccttttgc 6060

tggccttttg ctcacatgtt ctttcctgcg ttatcccctg attctgtgga taaccgtatt 6120

accgccatgc at 6132

<210> 2

<211> 6132

<212> DNA

<213> Artificial

<400> 2
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggtcgcca ccatggtgag caagggcgag gagctgttca ccggggtggt gcccatcctg 660

gtcgagctgg acggcgacgt aaacggccac aagttcagcg tgtccggcga gggcgagggc 720

gatgccacct acggcaagct gaccctgaag ttcatctgca ccaccggcaa gctgcccgtg 780

ccctggccca ccctcgtgac caccttcggc tacggcctgc agtgcttcgc ccgctacccc 840

gaccacatga agcagcacga cttcttcaag tccgccatgc ccgaaggcta cgtccaggag 900

cgcaccatct tcttcaagga cgacggcaac tacaagaccc gcgccgaggt gaagttcgag 960

ggcgacaccc tggtgaaccg catcgagctg aagggcatcg acttcaagga ggacggcaac 1020

atcctggggc acaagctgga gtacaactac aacagccaca acgtctatat catggccgac 1080

aagcagaaga acggcatcaa ggtgaacttc aagatccgcc acaacatcga ggacggcagc 1140

gtgcagctcg ccgaccacta ccagcagaac acccccatcg gcgacggccc cgtgctgctg 1200

cccgacaacc actacctgag ctaccagtcc gccctgagca aagaccccaa cgagaagcgc 1260

gatcacatgg tcctgctgga gttcgtgacc gccgccggga tcactctcgg catggacgag 1320

ctgtacaagt ccggagccat ggaaccaaag cggatcagag agggctacct tgtgaagaag 1380

gggagcgtgt tcaatacgtg gaaacccatg tgggttgtat tgttagaaga tggaattgaa 1440

ttctataaga agaaaagtga caacagcccc aaaggaatga tcccgctgaa agggagcact 1500

ctgactagcc cttgtcaaga ctttggcaaa aggatgtttg tgtttaagat cactacgacc 1560

aaacagcagg accacttctt ccaggcagcc ttcctggagg agagagatgc ctgggttcgg 1620

gatatcaata aggccattaa atgcattgaa ggaggccaga aatttgccag gaaagaagaa 1680

aggagggaaa ttcgactgcc agaaaccatt gacttaggtg ccttatattt gtccatgaaa 1740

gacactgaaa aaggaataaa agaactgaat ctagagaagg acaagaagat ttttaatcac 1800

tgcttcacag gtaactgcgt cattgattgg ctggtatcca accagtctgt taggaatcgc 1860

caggaaggcc tcatgattgc ttcatcgctg ctcaatgagg ggtatctgca gcctgctgga 1920

gacatgtcca agagtgcagt ggatggaact gctgaaaacc ctttcctgga caaccctgat 1980

gccttctact actttccaga cagtgggttc ttctgtgaag agaattccag tgatgatgat 2040

gtgattctga aagaactgcg ccgggccagc ctgggcggat ccatggtgag caagggcgag 2100

gagctgttca ccggggtggt gcccatcctg gtcgagctgg acggcgacgt aaacggccac 2160

aagttcagcg tgtccggcga gggcgagggc gatgccacct acggcaagct gaccctgaag 2220

ttcatctgca ccaccggcaa gctgcccgtg ccctggccca ccctcgtgac caccctgagc 2280

tacggcgtgc agtgcttcag ccgctacccc gaccacatga agcagcacga cttcttcaag 2340

tccgccatgc ccgaaggcta cgtccaggag cgcaccatct tcttcaagga cgacggcaac 2400

tacaagaccc gcgccgaggt gaagttcgag ggcgacaccc tggtgaaccg catcgagctg 2460

aagggcatcg acttcaagga ggacggcaac atcctggggc acaagctgga gtacaactac 2520

aacagccaca acgtctatat catggccgac aagcagaaga acggcatcaa ggtgaacttc 2580

aagatccgcc acaacatcga ggacggcagc gtgcagctcg ccgaccacta ccagcagaac 2640

acccccatcg gcgacggccc cgtgctgctg cccgacaacc actacctgag cacccagtcc 2700

gccctgagca aagaccccaa cgagaagcgc gatcacatgg tcctgctgga gttcgtgacc 2760

gccgccggga tcactctcgg catggacgag ctgtacaagt aatctagata actgatcata 2820

atcagccata ccacatttgt agaggtttta cttgctttaa aaaacctccc acacctcccc 2880

ctgaacctga aacataaaat gaatgcaatt gttgttgtta acttgtttat tgcagcttat 2940

aatggttaca aataaagcaa tagcatcaca aatttcacaa ataaagcatt tttttcactg 3000

cattctagtt gtggtttgtc caaactcatc aatgtatctt aaggcgtaaa ttgtaagcgt 3060

taatattttg ttaaaattcg cgttaaattt ttgttaaatc agctcatttt ttaaccaata 3120

ggccgaaatc ggcaaaatcc cttataaatc aaaagaatag accgagatag ggttgagtgt 3180

tgttccagtt tggaacaaga gtccactatt aaagaacgtg gactccaacg tcaaagggcg 3240

aaaaaccgtc tatcagggcg atggcccact acgtgaacca tcaccctaat caagtttttt 3300

ggggtcgagg tgccgtaaag cactaaatcg gaaccctaaa gggagccccc gatttagagc 3360

ttgacgggga aagccggcga acgtggcgag aaaggaaggg aagaaagcga aaggagcggg 3420

cgctagggcg ctggcaagtg tagcggtcac gctgcgcgta accaccacac ccgccgcgct 3480

taatgcgccg ctacagggcg cgtcaggtgg cacttttcgg ggaaatgtgc gcggaacccc 3540

tatttgttta tttttctaaa tacattcaaa tatgtatccg ctcatgagac aataaccctg 3600

ataaatgctt caataatatt gaaaaaggaa gagtcctgag gcggaaagaa ccagctgtgg 3660

aatgtgtgtc agttagggtg tggaaagtcc ccaggctccc cagcaggcag aagtatgcaa 3720

agcatgcatc tcaattagtc agcaaccagg tgtggaaagt ccccaggctc cccagcaggc 3780
agaagtatgc aaagcatgca tctcaattag tcagcaacca tagtcccgcc cctaactccg 3840
cccatcccgc ccctaactcc gcccagttcc gcccattctc cgccccatgg ctgactaatt 3900
ttttttattt atgcagaggc cgaggccgcc tcggcctctg agctattcca gaagtagtga 3960
ggaggctttt ttggaggcct aggcttttgc aaagatcgat caagagacag gatgaggatc 4020
gtttcgcatg attgaacaag atggattgca cgcaggttct ccggccgctt gggtggagag 4080
gctattcggc tatgactggg cacaacagac aatcggctgc tctgatgccg ccgtgttccg 4140
gctgtcagcg caggggcgcc cggttctttt tgtcaagacc gacctgtccg gtgccctgaa 4200
tgaactgcaa gacgaggcag cgcggctatc gtggctggcc acgacgggcg ttccttgcgc 4260
agctgtgctc gacgttgtca ctgaagcggg aagggactgg ctgctattgg gcgaagtgcc 4320
ggggcaggat ctcctgtcat ctcaccttgc tcctgccgag aaagtatcca tcatggctga 4380
tgcaatgcgg cggctgcata cgcttgatcc ggctacctgc ccattcgacc accaagcgaa 4440
acatcgcatc gagcgagcac gtactcggat ggaagccggt cttgtcgatc aggatgatct 4500
ggacgaagag catcaggggc tcgcgccagc cgaactgttc gccaggctca aggcgagcat 4560
gcccgacggc gaggatctcg tcgtgaccca tggcgatgcc tgcttgccga atatcatggt 4620
ggaaaatggc cgcttttctg gattcatcga ctgtggccgg ctgggtgtgg cggaccgcta 4680
tcaggacata gcgttggcta cccgtgatat tgctgaagag cttggcggcg aatgggctga 4740
ccgcttcctc gtgctttacg gtatcgccgc tcccgattcg cagcgcatcg ccttctatcg 4800
ccttcttgac gagttcttct gagcgggact ctggggttcg aaatgaccga ccaagcgacg 4860
cccaacctgc catcacgaga tttcgattcc accgccgcct tctatgaaag gttgggcttc 4920
ggaatcgttt tccgggacgc cggctggatg atcctccagc gcggggatct catgctggag 4980
ttcttcgccc accctagggg gaggctaact gaaacacgga aggagacaat accggaagga 5040
acccgcgcta tgacggcaat aaaaagacag aataaaacgc acggtgttgg gtcgtttgtt 5100
cataaacgcg gggttcggtc ccagggctgg cactctgtcg ataccccacc gagaccccat 5160
tggggccaat acgcccgcgt ttcttccttt tccccacccc accccccaag ttcgggtgaa 5220
ggcccagggc tcgcagccaa cgtcggggcg gcaggccctg ccatagcctc aggttactca 5280
tatatacttt agattgattt aaaacttcat ttttaattta aaaggatcta ggtgaagatc 5340
ctttttgata atctcatgac caaaatccct taacgtgagt tttcgttcca ctgagcgtca 5400
gaccccgtag aaaagatcaa aggatcttct tgagatcctt tttttctgcg cgtaatctgc 5460
tgcttgcaaa caaaaaaacc accgctacca gcggtggttt gtttgccgga tcaagagcta 5520
ccaactcttt ttccgaaggt aactggcttc agcagagcgc agataccaaa tactgtcctt 5580

ctagtgtagc cgtagttagg ccaccacttc aagaactctg tagcaccgcc tacatacctc 5640

gctctgctaa tcctgttacc agtggctgct gccagtggcg ataagtcgtg tcttaccggg 5700

ttggactcaa gacgatagtt accggataag gcgcagcggt cgggctgaac ggggggttcg 5760

tgcacacagc ccagcttgga gcgaacgacc tacaccgaac tgagatacct acagcgtgag 5820

ctatgagaaa gcgccacgct tcccgaaggg agaaaggcgg acaggtatcc ggtaagcggc 5880

agggtcggaa caggagagcg cacgagggag cttccagggg gaaacgcctg gtatctttat 5940

agtcctgtcg ggtttcgcca cctctgactt gagcgtcgat ttttgtgatg ctcgtcaggg 6000

gggcggagcc tatggaaaaa cgccagcaac gcggcctttt tacggttcct ggccttttgc 6060

tggccttttg ctcacatgtt ctttcctgcg ttatcccctg attctgtgga taaccgtatt 6120

accgccatgc at 6132

<210> 3

<211> 6132

<212> DNA

<213> Artificial

<400> 3
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggtcgcca ccatggtgag caagggcgag gagctgttca ccggggtggt gcccatcctg 660

gtcgagctgg acggcgacgt aaacggccac aagttcagcg tgtccggcga gggcgagggc 720

gatgccacct acggcaagct gaccctgaag ttcatctgca ccaccggcaa gctgcccgtg 780

ccctggccca ccctcgtgac caccttcggc tacggcctgc agtgcttcgc ccgctacccc 840

gaccacatga agcagcacga cttcttcaag tccgccatgc ccgaaggcta cgtccaggag 900

cgcaccatct tcttcaagga cgacggcaac tacaagaccc gcgccgaggt gaagttcgag 960

ggcgacaccc tggtgaaccg catcgagctg aagggcatcg acttcaagga ggacggcaac 1020

atcctggggc acaagctgga gtacaactac aacagccaca acgtctatat catggccgac 1080

aagcagaaga acggcatcaa ggtgaacttc aagatccgcc acaacatcga ggacggcagc 1140

gtgcagctcg ccgaccacta ccagcagaac acccccatcg gcgacggccc cgtgctgctg 1200

cccgacaacc actacctgag ctaccagtcc gccctgagca aagaccccaa cgagaagcgc 1260

gatcacatgg tcctgctgga gttcgtgacc gccgccggga tcactctcgg catggacgag 1320

ctgtacaagt ccggagccat ggaaccaaag cggatcagag agggctacct tgtgaagaag 1380

gggagcgtgt tcaatacgtg gaaacccatg tgggttgtat tgttagaaga tggaattgaa 1440

ttctataaga agaaaagtga caacagcccc aaaggaatga tcccgctgaa agggagcact 1500

ctgactagcc cttgtcaaga ctttggcaaa aggatgtttg tgtttaagat cactacgacc 1560

aaacagcagg accacttctt ccaggcagcc ttcctggagg agagagatgc ctgggttcgg 1620

gatatcaata aggccattaa atgcattgaa ggaggccaga aatttgccag gaaatctacc 1680

aggaggtcca ttcgactgcc agaaaccatt gacttaggtg ccttatattt gtccatgaaa 1740

gacactgaaa aaggaataaa agaactgaat ctagagaagg acaagaagat ttttaatcac 1800

tgcttcacag gtaactgcgt cattgattgg ctggtatcca accagtctgt taggaatcgc 1860

caggaaggcc tcatgattgc ttcatcgctg ctcaatgagg ggtatctgca gcctgctgga 1920

gacatgtcca agagtgcagt ggatggaact gctgaaaacc ctttcctgga caaccctgat 1980

gccttctact actttccaga cagtgggttc ttctgtgaag agaattccag tgatgatgat 2040

gtgattctga aagaactgcg ccgggccagc ctgggcggat ccatggtgag caagggcgag 2100

gagctgttca ccggggtggt gcccatcctg gtcgagctgg acggcgacgt aaacggccac 2160

aagttcagcg tgtccggcga gggcgagggc gatgccacct acggcaagct gaccctgaag 2220

ttcatctgca ccaccggcaa gctgcccgtg ccctggccca ccctcgtgac caccctgagc 2280

tacggcgtgc agtgcttcag ccgctacccc gaccacatga agcagcacga cttcttcaag 2340

tccgccatgc ccgaaggcta cgtccaggag cgcaccatct tcttcaagga cgacggcaac 2400

tacaagaccc gcgccgaggt gaagttcgag ggcgacaccc tggtgaaccg catcgagctg 2460

aagggcatcg acttcaagga ggacggcaac atcctggggc acaagctgga gtacaactac 2520

aacagccaca acgtctatat catggccgac aagcagaaga acggcatcaa ggtgaacttc 2580

aagatccgcc acaacatcga ggacggcagc gtgcagctcg ccgaccacta ccagcagaac 2640

acccccatcg gcgacggccc cgtgctgctg cccgacaacc actacctgag cacccagtcc 2700

gccctgagca aagaccccaa cgagaagcgc gatcacatgg tcctgctgga gttcgtgacc 2760

gccgccggga tcactctcgg catggacgag ctgtacaagt aatctagata actgatcata 2820

atcagccata ccacatttgt agaggtttta cttgctttaa aaaacctccc acacctcccc 2880

ctgaacctga aacataaaat gaatgcaatt gttgttgtta acttgtttat tgcagcttat 2940

aatggttaca aataaagcaa tagcatcaca aatttcacaa ataaagcatt tttttcactg 3000

cattctagtt gtggtttgtc caaactcatc aatgtatctt aaggcgtaaa ttgtaagcgt 3060

taatattttg ttaaaattcg cgttaaattt ttgttaaatc agctcatttt ttaaccaata 3120

ggccgaaatc ggcaaaatcc cttataaatc aaaagaatag accgagatag ggttgagtgt 3180

tgttccagtt tggaacaaga gtccactatt aaagaacgtg gactccaacg tcaaagggcg 3240

aaaaaccgtc tatcagggcg atggcccact acgtgaacca tcaccctaat caagtttttt 3300

ggggtcgagg tgccgtaaag cactaaatcg gaaccctaaa gggagccccc gatttagagc 3360

ttgacgggga aagccggcga acgtggcgag aaaggaaggg aagaaagcga aaggagcggg 3420

cgctagggcg ctggcaagtg tagcggtcac gctgcgcgta accaccacac ccgccgcgct 3480

taatgcgccg ctacagggcg cgtcaggtgg cacttttcgg ggaaatgtgc gcggaacccc 3540

tatttgttta tttttctaaa tacattcaaa tatgtatccg ctcatgagac aataaccctg 3600

ataaatgctt caataatatt gaaaaaggaa gagtcctgag gcggaaagaa ccagctgtgg 3660

aatgtgtgtc agttagggtg tggaaagtcc ccaggctccc cagcaggcag aagtatgcaa 3720

agcatgcatc tcaattagtc agcaaccagg tgtggaaagt ccccaggctc cccagcaggc 3780

agaagtatgc aaagcatgca tctcaattag tcagcaacca tagtcccgcc cctaactccg 3840

cccatcccgc ccctaactcc gcccagttcc gcccattctc cgccccatgg ctgactaatt 3900

ttttttattt atgcagaggc cgaggccgcc tcggcctctg agctattcca gaagtagtga 3960

ggaggctttt ttggaggcct aggcttttgc aaagatcgat caagagacag gatgaggatc 4020

gtttcgcatg attgaacaag atggattgca cgcaggttct ccggccgctt gggtggagag 4080

gctattcggc tatgactggg cacaacagac aatcggctgc tctgatgccg ccgtgttccg 4140

gctgtcagcg caggggcgcc cggttctttt tgtcaagacc gacctgtccg gtgccctgaa 4200

tgaactgcaa gacgaggcag cgcggctatc gtggctggcc acgacgggcg ttccttgcgc 4260

agctgtgctc gacgttgtca ctgaagcggg aagggactgg ctgctattgg gcgaagtgcc 4320

ggggcaggat ctcctgtcat ctcaccttgc tcctgccgag aaagtatcca tcatggctga 4380

tgcaatgcgg cggctgcata cgcttgatcc ggctacctgc ccattcgacc accaagcgaa 4440

acatcgcatc gagcgagcac gtactcggat ggaagccggt cttgtcgatc aggatgatct 4500

ggacgaagag catcaggggc tcgcgccagc cgaactgttc gccaggctca aggcgagcat 4560

gcccgacggc gaggatctcg tcgtgaccca tggcgatgcc tgcttgccga atatcatggt 4620

ggaaaatggc cgcttttctg gattcatcga ctgtggccgg ctgggtgtgg cggaccgcta 4680

tcaggacata gcgttggcta cccgtgatat tgctgaagag cttggcggcg aatgggctga 4740

ccgcttcctc gtgctttacg gtatcgccgc tcccgattcg cagcgcatcg ccttctatcg 4800

ccttcttgac gagttcttct gagcgggact ctggggttcg aaatgaccga ccaagcgacg 4860

cccaacctgc catcacgaga tttcgattcc accgccgcct tctatgaaag gttgggcttc 4920

ggaatcgttt tccgggacgc cggctggatg atcctccagc gcggggatct catgctggag 4980

ttcttcgccc accctagggg gaggctaact gaaacacgga aggagacaat accggaagga 5040

acccgcgcta tgacggcaat aaaaagacag aataaaacgc acggtgttgg gtcgtttgtt 5100

cataaacgcg gggttcggtc ccagggctgg cactctgtcg ataccccacc gagaccccat 5160

tggggccaat acgcccgcgt ttcttccttt tccccacccc accccccaag ttcgggtgaa 5220

ggcccagggc tcgcagccaa cgtcggggcg gcaggccctg ccatagcctc aggttactca 5280

tatatacttt agattgattt aaaacttcat ttttaattta aaaggatcta ggtgaagatc 5340

ctttttgata atctcatgac caaaatccct taacgtgagt tttcgttcca ctgagcgtca 5400

gaccccgtag aaaagatcaa aggatcttct tgagatcctt tttttctgcg cgtaatctgc 5460

tgcttgcaaa caaaaaaacc accgctacca gcggtggttt gtttgccgga tcaagagcta 5520

ccaactcttt ttccgaaggt aactggcttc agcagagcgc agataccaaa tactgtcctt 5580

ctagtgtagc cgtagttagg ccaccacttc aagaactctg tagcaccgcc tacatacctc 5640

gctctgctaa tcctgttacc agtggctgct gccagtggcg ataagtcgtg tcttaccggg 5700

ttggactcaa gacgatagtt accggataag gcgcagcggt cgggctgaac ggggggttcg 5760

tgcacacagc ccagcttgga gcgaacgacc tacaccgaac tgagatacct acagcgtgag 5820

ctatgagaaa gcgccacgct tcccgaaggg agaaaggcgg acaggtatcc ggtaagcggc 5880

agggtcggaa caggagagcg cacgagggag cttccagggg gaaacgcctg gtatctttat 5940

agtcctgtcg ggtttcgcca cctctgactt gagcgtcgat ttttgtgatg ctcgtcaggg 6000

gggcggagcc tatggaaaaa cgccagcaac gcggcctttt tacggttcct ggccttttgc 6060

tggccttttg ctcacatgtt ctttcctgcg ttatcccctg attctgtgga taaccgtatt 6120

accgccatgc at 6132

<210> 4

<211> 717

<212> DNA

<213> Artificial

<400> 4
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgagcta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaag 717

<210> 5

<211> 717

<212> DNA

<213> Artificial

<400> 5
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccttcggcta cggcctgcag tgcttcgccc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagct accagtccgc cctgagcaaa gaccccaacg agaagcgcga tcacatggtc 660

ctgctggagt tcgtgaccgc cgccgggatc actctcggca tggacgagct gtacaag 717